(19) Europäisches Patentamt
European Patent Office
Office européen des brevets

(11) Publication number: **0 421 819 A1**

# (12) EUROPEAN PATENT APPLICATION

(21) Application number: 90310965.0

(22) Date of filing: 05.10.90

(51) Int. Cl.[5]: C07D 405/04, A61K 31/70

(30) Priority: 06.10.89 US 417989

(43) Date of publication of application: 10.04.91 Bulletin 91/15

(84) Designated Contracting States: AT BE CH DE DK ES FR GB GR IT LI LU NL SE

(71) Applicant: THE WELLCOME FOUNDATION LIMITED
Unicorn House 160 Euston Road
London NW1 2BP(GB)

(72) Inventor: Koszalka, George Walter
117 Priestly Creek Drive
Chapel Hill, North Carolina, 27514(US)
Inventor: Burns, Charlene Louise
1418 North Duke Street
Durham North Carolina 27701(US)
Inventor: Krenitsky, Thomas Anthony
106 Laurel Hill Road
Chapel Hill, North Carolina 27514(US)
Inventor: Rideout, Janet Litster
3101 Morningside Drive
Raleigh, North Carolina 27607(US)

(74) Representative: Garrett, Michael et al
The Wellcome Research Laboratories Group
Patents and Agreements Langley Court
Beckenham Kent BR3 3BS(GB)

## (54) Therapeutic nucleosides.

(57) 6- Substituted 2′,3′-dideoxynucleosides for use in the treatment or prophylaxis of hepatitis B virus (HBV) infections and a specific novel compound, 6-(cyclopropylmethylamino)purine-9-β-D-2′,3′-dideoxyribo furanoside for use in the treatment of prophylaxis of HBV and human retrovirus such as Human Immunodeficiency Virus (HIV) infections and pharmaceutical formulations containing the latter compound.

EP 0 421 819 A1

## THERAPEUTIC NUCLEOSIDES

The present invention relates to 6-substituted 2′,3′-dideoxy nucleosides, pharmaceutically acceptable derivatives thereof, and their use in therapy, particularly for the treatment or prophylaxis of certain viral infections.

A group of viral pathogens of major worldwide importance are the hepatitis viruses, in particular hepatitis B virus (HBV). Clinical effects of infection with HBV range from headache, fever, malaise, nausea, vomiting, anorexia and abdominal pains. Replication of the virus is usually controlled by the immune response, with a course of recovery lasting weeks or months in humans, but infection may be more severe leading to persistent chronic liver disease. In "Viral Infections of Humans" (Second Edition, Ed., Evans, A.S. (1982) Plenum Publishing Corporation, New York), Chapter 12 describes in detail the etiology of viral hapatitis infections.

European Patent Specification No. 0206497 describes certain purine 2,3′-dideoxynucleosides including 6-methylthiopurine-9-β-D-2′,3′-dideoxyribofuranoside and 6-methoxypurine-9-β-D-2′,3′-dideoxy ribofuranoside, and their use in the treatment or prophylaxis of viral infections.

European Patent Specification No. 0286425 describes certain 6-substituted purine 2′,3′-dideoxynucleosides and their use in the treatment or prophylaxis of Human Immunodeficiency Virus (HIV) infections.

We have now discovered that 6-substituted 2′,3′-dideoxynucleosides described in European Patent Specification No. 0286425 are effective against hepatitis B virus infections. We have further discovered a certain 6-substituted 2′,3′-dideoxynucleoside falling within the sccpe of the compounds broadly described in European Patent Specification No. 0286425, but not specifically described therein, has potent activity against Human Immunodeficency Virus (HIV), the causative agent of AIDS, as well as activity against hepatitis B virus infections.

The above-mentioned 6-substituted 2′,3′- dideoxynucleosides may be represented by the following general formula (I)

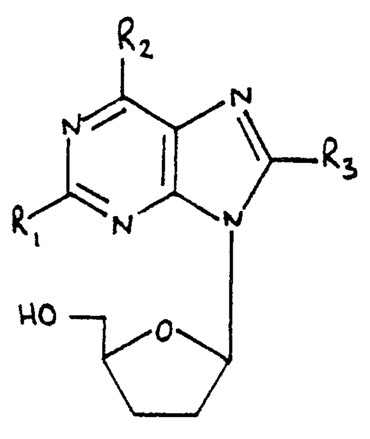

(I)

wherein $R_1$ represents hydrogen or amino; and $R_2$ represents halogen (e.g. chlorine), $C_{1-6}$ alkoxy (e.g. propyloxy or isopropoxy), optionally substituted for example by $C_{3-6}$ cycloalkyl (e.g. cyclopropylmethoxy); $C_{3-8}$ cycloalkyloxy (e.g. cyclobutyloxy or cyclopentyloxy); aryloxy (e.g. phenyloxy); aralkyl (e.g. benzyl) or aralkyloxy (e.g. benzyloxy) in which the aryl may optionally be substituted with lower alkyl, hydroxyl or halogen; $C_{3-6}$ cycloalkylthio; $C_{1-6}$ alkylthio; arylthio, or aralkylthio in which the aryl may optionally be substituted with lower alkyl, hydroxy, or halogen; or $R_2$ represents a heterocyclic group containing an oxygen atom or one or two nitrogen atoms, and 3-7 carbon atoms with optional double bonds in the ring (e.g. piperidino, pyrrolidino or furfuryl) optionally containing a sulphur and/or oxygen heteroatom and optionally substituted on the ring by one or more lower alkyl, hydroxyl or halogen groups, $C_{3-6}$ cycloalkylthio, aralkylthio in which the aryl may be substituted with lower alkyl, hydroxy or halogen; or $R_2$ represents an imidazolylthio group in which the imidazolyl moiety may be substituted with lower alkyl and/or C-substituted with nitro; or $R_2$ represents an amino group which is mono or di-substituted by $C_{1-6}$ alkyl (e.g. methyl or ethyl), $C_{1-6}$ alkoxy (e.g. methoxy), hydroxy $C_{1-6}$ alkyl (e.g. hydroxyethyl) and/or $C_{3-6}$ cycloalkyl (e.g. cyclopropyl or cyclopentyl), aryl (e.g. phenyl), aralkyl (e.g. benzyl) in which the aryl may optionally be substituted with lower alkyl, hydroxy or halogen, allyl optionally substituted with mono- or di-alkyl or alkoxy groups (e.g. dimethylallyl); and $R_3$ represents hydrogen or amino, and pharmaceutically acceptable derivatives thereof, other than the compounds of formula (I) in which $R_1$ and $R_3$ represent hydrogen and $R_2$ represents a methoxy or methylthio group.

According to the present invention, we provide the use of a compound of formula (I) above or a pharmaceutically acceptable derivative thereof in the manufacture of a pharmaceutical formulation for the treatment or prophylaxis of a hepatitis B virus infections.

The above references to "lower alkyl" denote groups containing 1 to 6 carbon atoms preferably methyl or ethyl. The references to halogen include chlorine, bromine, iodine and fluorine, chlorine and iodine being particularly preferred.

Preferred classes of the compounds of formula (I) include those in which $R_1$, $R_2$ and $R_3$ independently represent the following groups, namely $R_1$ represents amino; $R_3$ represents hydrogen; and $R_2$ represents a mono or di-substituted amino group, for example an amino group mono-and/or di-substituted by $C_{1-6}$ alkyl and/or $C_{3-6}$ cycloalkyl group; or a $C_{1-6}$ alkoxy group.

The following compounds are examples of compounds of formula (I) above:-

1. 6-N-Piperidinopurine-9-β-D-2′,3′-dideoxyribofuranoside
2. 6-Chloropurine-9-β-D-2′,3′-dideoxyribofuranoside
3. 6-Ethylaminopurine-9-β-D-2′,3′-dideoxyribofuranoside
4. 6-Ethylmethylamino-9-β-D-2′,3′-dideoxyribofuranoside
5. 6-Iodopurine-9-β-D-2′,3′-dideoxyribofuranoside
6. 6-cyclopropylmethylaminopurine-9-β-D-2′,3′-dideoxyribofuranoside
7. 6-Isopropylaminopurine-9-β-D-2′,3′-dideoxyribofuranoside
8. Thiamiprine-9-β-D-2′,3′-dideoxyribofuranoside
9. 2-Amino-6-n-propoxypurine-9-β-2′,3′-dideoxyribofuranoside
10. 6-Ethylthiopurine-9-β-D-2′,3′-dideoxyribofuranoside
11. 2-Amino-6-n-benzylthiopurine-9-β-D-2′,3′-dideoxyribofuranoside
12. 6-Ethoxypurine-9-β-D-2′,3′-dideoxyribofuranoside
13. 6-Dimethylaminopurine-9-β-D-2′,3′-dideoxyribofuranoside
14. 6-Hydroxyethylaminopurine-9-β-D-2′,3′-dideoxyribofuranoside
15. 6-Cyclopropylaminopurine-9-β-D-2′,3′-dideoxyribofuranoside
16. 6-Cyclopentylaminopurine-9-β-D-2′,3′-dideoxyribofuranoside
17. 2-Amino-6-methoxypurine-9-β-D-2′,3′-dideoxyribofuranoside
18. 6-n-Propoxypurine-9-β-D-2′,3′-dideoxyribofuranoside
19. 6-n-Butoxypurine-9-β-D-2′,3′-dideoxyribofuranoside
20. 6-Cyclopropylmethoxypurine-9-β-D-2′,3′-dideoxyribofuranoside
21. 6-Cyclopentyloxypurine-9-β-D-2′,3′-dideoxyribofuranoside
22. 6-Cyclohexyloxypurine-9-β-D-2′,3′-dideoxyribofuranoside
23. 6-Cyclobutylaminopurine-9-β-D-2′,3′-dideoxyribofuranoside
24. 6-Diethylaminopurine-9-β-D-2′,3′-dideoxyribofuranoside
25. 6-Pyrrolidinopurine-9-β-D-2′,3′-dideoxyribofuranoside
26. 6-Morpholinopurine-9-β-D-2′,3′-dideoxyribofuranoside
27. 6-Dimethylallylaminopurine-9-β-D-2′,3′-dideoxyribofuranoside
28. 6-Furfurylaminopurine-9-β-D-2′,3′-dideoxyribofuranoside
29. 6-Benzylmercaptopurine-9-β-D-2′,3′-dideoxyribofuranoside
30. 6-Anilinopurine-9-β-D-2′,3′-dideoxyribofuranoside
31. 2-Amino-6-ethoxypurine-9-β-D-2′,3′-dideoxyribofuranoside
32. 2,6,8-Triaminopurine-9-β-D-2′,3′-dideoxyribofuranoside
33. 2-Amino-6-benzylaminopurine-9-β-D-2′,3′-dideoxyribofuranoside
34. 2-Amino-6-cyclopropylaminopurine-9-β-D-2′,3′-dideoxyribofuranoside
35. 2-Amino-6-methylaminopurine-9-β-D-2′,3′-dideoxyribofuranoside
36. 6-Benzylaminopurine-9-β-D-2′,3′-dideoxyribofuranoside
37. 6-Isopropoxypurine-9-β-D-2′,3′-dideoxyribofuranoside
38. 6-Propylaminopurine-9-β-D-2′,3′-dideoxyribofuranoside
39. 6-Cyclohexylamino-9-β-D-2′,3′-dideoxyribofuranoside
40. 6-Methylaminopurine-9-β-D-2′-3′-dideoxyribofuranoside
41. 2-Amino-6-(cyclopropylmethylamino)purine-9-β-D-2′,3′-dideoxyribofuranoside

Compounds 9, 34 and 41 above are particularly preferred on account of their surprisingly high anti-HBV activity.

Compound 41, i.e. 2-amino-6-(cyclopropylmethylamino)purine-9-β-D-2′,3′-dideoxyribofuranoside and its

pharmaceutically acceptable derivatives are new compounds and represent a further feature of the present invention. The invention also includes the following:-

a) the above-defined novel compounds for use in medical therapy for example in the treatment or prophylaxis of viral infections for example human retrovirus such as HIV infections or HBV infections; and

b) pharmaceutical formulations comprising an above-defined novel compound together with at least one pharmaceutically acceptable carrier therefor.

Compound 41 has been found to possess exceptionally potent activity against HBV and HIV infections.

Examples of retroviral infections which may be treated with the above novel compounds in accordance with the invention include human retroviral infections such as human immunodeficiency virus (HIV), e.g. HIV-1 or HIV-2 and human T-cell lymphotropic virus (HLTV), e.g. HTLV-I or HTLV-II infections. The compounds according to the invention are especially useful for the treatment of AIDS and related clinical conditions such as AIDS- related complex (ARC), progressive generalized lymphadenopathy (PGL), AIDS-related neurological conditions, such as multiple sclerosis or tropical papaparesis, anti-HIV antibody-positive and HIV-positive conditions, such as thrombocytopenia purpura. The compounds may also be used in the treatment of psoriasis.

The novel compounds according to the invention have been found to be particularly applicable to the treatment of asymptomatic infections or diseases in humans caused by or associated with human retroviruses.

The compounds of formula (I) above and their pharmaceutically acceptable derivatives, are hereinafter referred to as the compounds according to the invention.

By "a pharmaceutically acceptable derivative" is meant any pharmaceutically acceptable salt, ester, or salt of such ester, of a compound according to the invention or any other compound which, upon administration to the recipient, is capable of providing (directly or indirectly) a compound according to the invention, or an antivirally active metabolite or residue thereof.

Preferred esters of the compounds of the invention include carboxylic acid esters in which the non-carbonyl moiety of the ester grouping is selected from straight or branched chain alkyl e.g. n-propyl, t-butyl, n-butyl, alkoxyalkyl (e.g. methoxymethyl), aralkyl (e.g. benzyl), aryloxyalkyl (e.g. phenoxymethyl), aryl (e.g. phenyl optionally substituted by halogen, $C_{1-4}$ alkyl or $C_{1-4}$ alkoxy); sulphonate esters such as alkyl- or aralkylsulphonyl (e.g. methanesulphonyl); amino acid esters (e.g. L-valyl or L-isoleucyl); and mono-, di- or tri-phosphate esters.

With regard to the above-described esters, unless otherwise specified, any alkyl moiety present advantageously contains 1 to 18 carbon atoms, particularly 1 to 4 carbon atoms. Any aryl moiety present in such esters advantageously comprises a phenyl group.

Any reference to any of the above compounds also includes a reference to a pharmaceutically acceptable salt thereof.

Examples of pharmaceutically acceptable salts of the compounds according to the invention and pharmaceutically acceptable derivatives thereof include base salts, e.g. derived from an appropriate base, such as alkali metal (e.g. sodium), alkaline earth metal (e.g. magnesium) salts, ammonium and $NX_4^+$ (wherein X is $C_{1-4}$ alkyl). Pharmaceutically acceptable salts of a hydrogen atom or an amino group include salts of organic carboxylic acids such as acetic, lactic, tartaric, malic, isethionic, lactobionic and succinic acids; organic sulfonic acids such as methanesulfonic, ethanesulfonic, benzenesulfonic and p-toluenesulfonic acids and inorganic acids such as hydrochloric, sulfuric, phosphoric and sulfamic acids. Pharmaceutically acceptable salts of a compound with a hydroxy group include the anion of said compound in combination with a suitable cation such as $Na^+$, $NH_4^+$, and $NX_4^+$ (wherein X is a $C_{1-4}$ alkyl group).

Specific examples of pharmaceutically acceptable derivatives of the compound of formula (I) that may be used in accordance with the present invention include the monosodium salt and the following 5′ esters: monophosphate; disodium monophosphate; diphosphate; triphosphate; acetate; 3-methyl-butyrate; octanoate; palmitate; 3-chloro benzoate; benzoate; 4-methyl benzoate; hydrogen succinate; pivalate; propionate; valerate and mesylate.

The above compounds according to the invention and their pharmaceutically acceptable derivatives may be employed in combination with other therapeutic agents for the treatment or prophylaxis of the above infections or conditions. The component compounds of such combination therapy may be administered simultaneously, in either separate or combined formulations, or at different times, e.g. sequentially such that a combined effect is achieved.

The compounds according to the invention, also referred to herein as the active ingredient, may be administered for therapy by any suitable route including oral, rectal, nasal, topical (including buccal and sublingual), vaginal and parenteral (including subcutaneous, intramuscular, intravenous and intradermal). It will be appreciated that the preferred route will vary with the condition and age of the recipient, the nature

of the infection and the chosen active ingredient.

In general a suitable dose will be in the range of 3.0 to 120 mg per kilogram body weight of the recipient per day, preferably in the range of 6 to 90 mg per kilogram body weight per day and most preferably in the range 15 to 60 mg per kilogram body weight per day. The desired dose is preferably presented as two, three, four, five, six or more sub-doses administered at appropriate intervals throughout the day. These sub-doses may be administered in unit dosage forms, for example, containing 10 to 1500 mg, preferably 20 to 1000 mg, and most preferably 50 to 700 mg of active ingredient per unit dosage form.

Ideally, the active ingredient should be administered to achieve peak plasma concentrations of the active compound of from about 1 to about 75 $\mu$M, preferably about 2 to 50 $\mu$M, most preferably about 3 to about 30 $\mu$M. This may be achieved, for example, by the intravenous injection of a 0.1 to 5% solution of the active ingredient, optionally in saline, or orally administered as a bolus containing about 1 to about 100 mg/kg of the active ingredient. Desirable blood levels may be maintained by a continuous infusion to provide about 0.01 to about 5.0 mg/kg/hour or by intermittent infusions containing about 0.4 to about 15 mg/kg of the active ingredient.

While it is possible for the active ingredient to be administered alone it is preferable to present it as a pharmaceutical formulation. The formulations of the present invention comprise at least one active ingredient, as defined above, together with at least one pharmaceutically acceptable carrier therefor and optionally other therapeutic agents. Each carrier must be "acceptable" in the sense of being compatible with the other ingredients of the formulation and not injurious to the patient. Formulations include those suitable for oral, rectal, nasal, topical (including buccal and sublingual), vaginal or parenteral (including subcutaneous, intramuscular, intravenous and intradermal) administration. The formulations may conveniently be presented in unit dosage form and may be prepared by any methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then if necessary shaping the product.

Formulations of the present invention suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, cross-linked sodium carboxymethyl cellulose) surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile. Tablets may optionally be provided with an enteric coating, to provide release in parts of the gut other than the stomach. This is particularly advantageous for purine nucleoside derivatives as such compounds are susceptible to acid hydrolysis.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non- aqueous isotonic sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example, ampoules and vials, and may be stored in a freeze-dried (lyophilized) condition requiring only the addition of the sterile liquid carrier, for example

water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above recited, or an appropriate fraction thereof, of an active ingredient.

The compounds according to the invention may also be presented for use in the form of veterinary formulations, which may be prepared, for example, by methods that are conventional in the art.

It should be understood that in addition to the ingredients particularly mentioned above the formulations of this invention may include other agents conventional in the art having regard to the type of formulation in question, for example, those suitable for oral administration may include such further agents as sweeteners, thickeners and flavouring agents.

The present invention further includes a process for the preparation of a compound according to the invention and pharmaceutically acceptable derivatives thereof which comprises either:

(A) reacting a compound of formula:

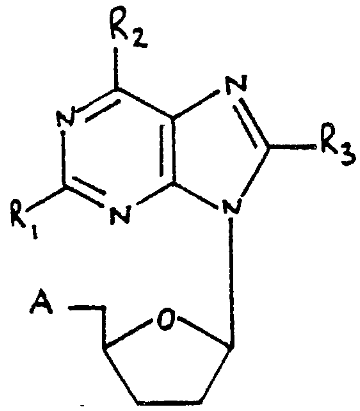

(II)

(wherein $R_1$, $R_2$ and $R_3$ are as hereinbefore defined and A represents a precursor group for the hydroxy group, or for a pharmaceutically acceptable derivative group thereof) with an agent or under conditions serving to convert the said precursor group into the corresponding desired group; or

(B) reacting a purine base of formula

B - H (III)

(wherein B is the required purine moiety of a compound according to the invention).

or a functional equivalent thereof, with a compound serving to introduce the desired ribofuranosyl ring at the 9- position of the purine base of formula (III);

and thereafter, or simultaneously therewith, effecting one or more of the following optional conversions:-

(i) when a compound of formula (I) is formed, converting it into a pharmaceutically acceptable derivative thereof,

(ii) when a pharmaceutically acceptable derivative of a compound of formula (I) is formed, converting the said derivative into a compound of formula (I), or a different derivative thereof.

In the above-described process according to the invention, it will be appreciated that the precursor compounds of formula (I) as well as the above-mentioned agents and conditions, will be selected from those that are known in the art of nucleoside synthetic chemistry. Examples of such conversion procedures are described hereinafter for guidance and it will be understood that they can be modified in conventional manner depending on the desired compound of formula (I). In particular, where a conversion is described which would otherwise result in the undesired reaction of labile groups then such groups may be protected in conventional manner, with subsequent removal of the protecting groups after completion of the conversion.

With regard to process (A), A may represent a protected hydroxy group e.g. an ester grouping of the type referred to above in relation to formula (I) particularly acetoxy, or an ether group such as a trialkylsilyloxy group, e.g. t- butyldimethylsilyloxy or an aralkoxy group e.g. triphenylmethoxy. Such groups may be converted for example by hydrolysis to the desired hydroxy group or, by transesterification, to an alternative ester group.

With regard to process (B), this may be effected for example by treating an appropriate purine base of formula (III) or a salt or protected derivative thereof, with 3′-deoxythymidine for example in the presence of the appropriate pentosyl transferring enzyme.

A compound of formula (I) may be converted into a pharmaceutically acceptable phosphate or other ester by reaction with respectively a phosphorylating agent, e.g. $POCl_3$ or an appropriate esterifying agent, e.g. an acid halide or anhydride. The compound of formula (I), including esters thereof, may be converted into pharmaceutically acceptable salts thereof in conventional manner, e.g. by treatment with an appropriate base. An ester or salt of a compound of formula (I) may be converted into the parent compound, e.g. by hydrolysis.

The following Examples are intended for illustration only and are not intended to limit the scope of the invention in any way. The term 'active ingredient' as used in the Examples means a compound of formula (I) or a pharmaceutically acceptable derivative thereof.

Example I

6-N-Piperidinopurine-9-β-D-2′,3′-dideoxyribofuranoside

6-N-Piperidinopurine (2.41 mmol, 0.5g, Sigma Chemicals, St. Louis MO) was dissolved in 10ml of dimethylsulfoxide with heat. After cooling to room temperature 3′-deoxythymidine (3.62mmol, 0.82g)-(Howitz,J.P. et al , J. Org. Chem. 31 , 205 (1966)) was added along with 30ml of 10mM potassium phosphate buffer with a pH of 6.8 containing 0.04% potassium azide.

Purified thymidine phosphorylase (10,000 I. U.) and purine nucleoside phosphorylase (20,000 I. U.) (Krenitsky T.A. et al ., Biochemistry , 20 , 3615, 1981 and US Patent 4,381,344) adsorbed onto 10ml of DEAE cellulose (Whatman) were added, and the suspension was stirred at 35°C. After 8 hours the reaction was filtered, and the filtrate was applied to a series of coupled columns. The initial column contained AG1 X2 hydroxide resin (2.5 x 10cm) while the second column was filled with Amberlite XAD-2 resin (2.5 x 20cm). After sample application, the columns were washed with a large volume of water and the product was eluted with methanol. After removal of the solvent and redissolving in chloroform:methanol (9:1, v/v), additional chromatography was performed on a column containing silica gel (5 X 20cm). The mobile phase was chloroform:methanol (9:1, v/v). Product containing fractions were combined, redissolved in ethanol, and filtered through a 0.22 filter. The ethanol was evaporated, and the product was redissolved in water. After lyophilization, the 6-N-piperidinopurine-9-β-D-2′,3′- dideoxyribofuranoside (0.265g) analyzed as a 0.1 hydrate containing 0.3 ethanol.

| Anal. Calcd. for $C_{15}H_{21}N_5O_2$ 0.3 $C_2H_6O$: | | | |
|---|---|---|---|
| Calcd.: | C, 58.74; | H, 7.27; | N, 21.96 |
| Found: | C, 58.86; | H, 7.14; | N, 21.82 |

NMR: 8.36 (s, 1 H, $H_8$), 8.19 (s, 1 H, $H_2$), 6.23(dd, 1 H, $H_{1'}$), 5.01 (t, 1 H, J = 5.54, $OH_{5'}$), 4.12 (m, 3 H, $H_{4'}$, $CH_2$), 3.52 (m, 2 H, $H_{5'}$), 2.37 (m, 2 H, $H_{2'}$), 2.04 (m, 2 H, $H_{3'}$), 1.61 (b, 6 H, 3 $CH_2$).

Example 2

6-Chloropurine-9-β-D-2′,3′-dideoxyribofuranoside

The synthesis of 6-chloropurine-9-β-D -2′,3′-dideoxyribofuranoside was performed as described in Example 1 except that the 6-chloropurine (Sigma Chemicals, St. Louis Mo) was dissolved in 5ml each of dimethylformamide and dimethoxyethane.

After filtering off the solids, the filtrate was reduced to ~5ml under vacuum then dissolved in 100ml water. This material was chromatographed on a 2.5 X 20 cm column containing XAD-2 resin. After washing this column with 500ml of water, the product was eluted with methanol. Product containing fractions were combined and 20ml of dry silica gel added. All solvent was removed under vacuum. The dry silica gel was applied to the top of a silica gel column equilibrated with chloroform:methanol (9:1, v/v). Product containing fractions free of deoxythymidine were combined, and after removal of the solvent under vacuum, the residue was dissolved in ethanol, filtered, then dissolved in water and lyophilized. This material was further

purified by chromatography on a column containing Polygosil $C_{18}$ resin in methanol:water (8:2, v/v). After removal of the solvent in vacuo, the product was dissolved in water and lyophilized yielding 0. 199g of 6-chloropurine-9-β-D-2′,3′-dideoxyribofuranoside (mp = 100°C).

| Anal. Calcd. for $C_{10}H_{11}ClN_4O_2$: | | | | |
|---|---|---|---|---|
| Calcd.: | C, 47.16; | H, 4.35; | N, 22.00; | Cl, 13.92 |
| Found: | C, 47.10; | H, 4.35 | N, 21.94; | Cl, 13.86 |

Example 3

6-Ethylaminopurine-9-β-D-2′,3′-dideoxyribofuranoside

6-Ethylaminopurine (prepared by nucleophilic displacement of the chlorine group on 6-chloropurine (Sigma Chemicals, St. Louis Mo) by the amino group of ethylamine) (2.69mmol, 0.5g) and 3′-deoxythymidine (Horwitz, J.P. et al , J.Org.Chem ., 31 205 (1966)) (3.33mmol, 0. 755g) were combined along with 50ml of 10mM potassium phosphate buffer with a pH of 6.8, containing 0.04% potassium azide. Purified thymidine phosphorylase (400 I. U.) and purine nucleoside phosphorylase (700 I. U.) were added and the suspension was stirred at 37°C. After 48 hours an additional 700 units of purine nucleoside phosphorylase and 400 units of thymidine phosphorylase were added, and the reaction was stirred at 37°C. Five days later the reaction was filtered, and the filtrate was applied to a column containing AG-1 X2 hydroxide resin (2.5 x 10cm). The product was eluted with a water wash and chromatographed on Amberlite XAD-2 resin (2.5 x 20cm). After sample application, this column was washed with a large volume of water. The product was eluted with methanol. After removal of the solvent, the product was redissolved in water and acetone then lyophilized yielding 0.299g of 6-ethylaminopurine-9-β-D-2′,3′-dideoxyribofuranoside that analyzed for 0.2 water and 0.1 acetone (mp = < 30°C, [α]20°C =-29.45°C (0.5, DMF)).

| Anal. Calcd. for $C_{12}H_{17}N_5O_2$ 0.2 $H_2O$ 0.1 $C_3H_6O$: | | | |
|---|---|---|---|
| Calcd.: | C, 54.17; | H, 6.64; | N, 25.68 |
| Found: | C, 54.13; | H, 6.69; | N, 25.75 |

Example 4

6-Ethylmethylaminopurine-9-β-D-2′,3′-dideoxyribofuranoside

The procedure for the synthesis of 6-ethylmethylaminopurine-9-β-D-2′,3′-dideoxyribofuranoside was identical to Example 2. The reaction was filtered and the filtrate applied to a Dowex-1-hydroxide column (2.5 x 10cm). The product was eluted with 90% methanol/water (v/v) and chromatographed on Amberlite XAD-2 resin (2.5 x 20cm) after removal of the solvent to ~5ml and redissolving in water (100ml). After sample application, the column was washed with a large volume of water, and the product was eluted with 95% ethanol/water (v/v). Product containing fractions were combined and 20ml of dry silica gel added. All solvent was removed under vacuum. The dried silica gel was applied to the top of a silica gel column (4.8 X 20cm) equilibrated with chloroform:methanol (98:2, v/v). Product containing fractions were combined and after removal of the solvent under vacuum, were dissolved first in ethanol and filtered. After removal of the solvent and redissolving in water, the solution was lyophilized yielding 0.3g of 6-ethylmethylamino-9-β-D-2′,3′-dideoxyribofuranosylpurine that analyzed for a 0.05 hydrate (mp < 30°C).

| Anal. Calcd. for $C_{13}H_{19}N_5O_2$ 0.05 $H_2O$: | | | |
|---|---|---|---|
| Calcd.: | C, 56.12; | H, 6.92; | N, 25.17 |
| Found: | C, 56.12; | H, 6.94; | N, 25.14 |

NMR: δ 8.36 (s, 1 H, $H_8$), 8.19 (s, 1 H, $H_2$), 6.23 (dd, 1 H, $H_{1'}$), 5.05 (t, 1H, J = 5.58, $OH_{5'}$), 4.09 (m, 1, H, $H_{4'}$), 4.08 (m, 2 H, $CH_2$), 3.51 (m, 2 H, $H_{5'}$), 3.33 (s, 3 H, $CH_3$), 2.41 (m, 2 H, $H_{2'}$), 2.03 (m, 2 H, $H_{3'}$), 1.14 (t, 3 H, J = 7.01, $CH_3$).

Example 5

6-Iodopurine-9-β-D-2′,3′-dideoxyribofuranoside

6-Iodopurine (0. 624g 2.54mmol, Sigma Chemicals, St. Louis MO) and 3′-deoxythymidine (0.71g, 3.13mmol) (Horwitz JP et al J.Org.Chem ., 31 , 205 (1966)) were combined with 700ml 10 mM potassium phosphate buffer with a pH of 6.8, containing 0.04% potassium azide. Purified thymidine phosphorylase (2,000 I.U.) and purine nucleoside phosphorylase (7,000 I. U.) (Krenitsky T.A., et al ., Biochemistry , 20 , 3615, 1981 and US Patent 4,381,344) were added and the suspension was stirred at 35°C. After 48 hours the reaction was filtered, and the filtrate was dried under vacuum. The resulting residue was dissolved in 95% ethanol/water (v/v), and after adding ~20ml silica gel, the solvent was removed under vacuum. The dried silica was applied to the top of a silica gel column (2.8 X 50cm) and the product eluted with chloroform/methanol (95:5, v/v). Fractions containing only product were combined, and the solvent was removed under vacuum. The residue was redissolved in ethanol and filtered through a 0.22 filter. After removing most of the ethanol and adding ~25ml of water, the material was lyophilized yielding 0.088g of 6-iodopurine-9-β-D-2′,3′-dideoxyribofuranoside that analyzed as a 0.2 hydrate (mp = 151-153°C).

| Anal. Calcd. for $C_{10}H_{11}N_4O_2$ 0.2 $H_2O$: | | | |
|---|---|---|---|
| Calcd.: | C, 35.15; | H, 3.46; | N, 15.77 |
| Found: | C, 35.31; | H, 3.31 | N, 15.83 |

Example 6

6-Cyclopropylmethylaminopurine-9-β-D-2′,3-dideoxyribofuranoside

6-Cyclopropylmethylaminopurine was prepared by nucleophilic displacement of the chlorine group on 6-chloropurine (Sigma Chemicals. St. Louis MO) by the amino group on cyclopropylmethylamine (Karl Industries, Aurora, OH).

6-Cyclopropylmethylaminopurine (2.64 mmol 0.50 g) was dissolved in 5 ml of dimethylformamide. After cooling to room temperature 3′-deoxythymidine (3.98 mmol, 0.90 g) (Horwitz, J.P. et al ., J . Org . Chem . 31 , 205 (1966)) was added along with 30 ml of 10 mM potassium phosphate buffer with a pH of 6.8 containing 0.04% potassium azide. Purified thymidine phosphorylase (10,000 I.U.) and purine nucleoside phosphorylase (20,000 I.U) (Krenitsky T.A. et al ., Biochemistry 20 , 3615, 1981 and US Patent 4,381,344) absorbed onto 10 ml DEAE cellulose (Whatman) were added, and the suspension was stirred at 35°C. After 8 hours the reaction was filtered, and the filtrate was applied to a series of coupled columns. The initial column contained AG1-X2 resin (OH-form), 2.5 x 10 cm, while the second column contained Amberlite XAD-2 resin, 2.5 x 20 cm. After sample application, the columns were washed with 500 ml water and the product was eluted with methanol. The product was then flash chromatographed on a silica gel column, 5 x 20 cm, with a mixture of chloroform:methanol (9:1, v/v). Solvent was removed in vacuo and the product gum was transferred in acetone to a vial. Lyophilisation yielded 0.588 g of 6-cyclopropylmethyalaminopurine-9-β-D-2',3'-dideoxyribofuranoside that analysed for 0.15 water and 0.15 acetone.

| Anal. Calcd. for $C_{14}H_{19}N_5O_2$ 0.15 $H_2O$ 0.15 $C_3H_6O$: | | | |
|---|---|---|---|
| Calcd.: | C, 57.71; | H, 6.77; | N, 23.29 |
| Found: | C, 57.73; | H, 6.94; | N, 23.39 |

Example 7

6-Isopropylaminopurine-9-β-D-2',3'-dideoxyribofuranoside

The synthesis of 6-isopropylaminopurine-9-β-D-2',3'-dideoxyribofuranoside was performed as described in Example 1 except that 6-isopropylaminopurine (prepared from 6-chloropurine (Sigma Chemicals, St. Louis MO) and isopropylamine) was dissolved in 5ml each of dimethylformamide and dimethylsulfoxide.

After lyophilization, the 6-isopropylaminopurine-9-β-D-2',3'-dideoxyribofuranoside (0.502g) analyzed for 0.2 hydrate (mp = 55-57°C).

| Anal. Calcd. for $C_{13}H_{19}N_5O_2$ 0.2 $H_2O$: | | | |
|---|---|---|---|
| Calcd.: | C, 55.58; | H, 6.96; | N, 24.93 |
| Found: | C, 55.54; | H, 6.96; | N, 25.01 |

Example 8

Thiamiprine-9-β-D-2',3'-dideoxyribofuranoside

Thiamiprine (Burroughs Wellcome Co., Research Triangle Park NC) (0.5g) was dissolved in 2.5ml dimethylsulfoxide and 15ml dimethoxyethane and combined with 3'-deoxythymidine (0.8g) (Horwitz J.P. et al J.Org . Chem , 31 , 205, (1966)) in 30ml potassium phosphate pH 6.8. Purified thymidine phosphorylase (1600 I.U.) and purine nucleoside phosphorylase (70,000 I.U.) (Krenitsky T.A., et al ., Biochemistry , 20 , 3615, 1981 and US Patent 4,381,344) were added and the suspension was stirred at 35°C. After 96 hours the reaction was filtered and the volume reduced in vacuo to a syrup. Water (25ml) was added and the solution stored overnight at 3°C. The precipitate was collected by filtration, suspended in 5ml dimethylformamide and filtered. To the filtrate was added 15ml methanol, and the solution was stored at -20°C. After 5 days the solids were collected by filtration, dissolved in 65% methanol/water (v/v) and chromatograhed on a AG-1 X2 hydroxide resin. The product was eluted with 65% methanol/water (v/v). After removal of the solvent in vacuo, the solids were dissolved in 20ml chloroform/methanol (9:1) and chromatographed on a bed of silica gel (3 x 50cm) equilibrated with chloroform/methanol (9:1, v/v). Product containing fractions were combined and the solvent removed under vacuum. The residual silica gel was removed from the product by dissolving in 95% ethanol/water (v/v) and filtering through a 0.22 filter. The ethanol was evaporated off and ~200ml water were added. The resulting suspension was lyophilized yielding 0.056g Thiamiprine-9-β-D-2',3'-dideoxyribofuranoside that analyzed as a 0.4 hydrate containing 0.7 equivalents of methanol (mp = 130°C, partial melting at 110°C).

| Anal. Calcd. for $C_{14}H_{16}SN_8O_4$0.4 $H_2O$ 0.7 $CH_4O$: | | | | |
|---|---|---|---|---|
| Calcd.: | C, 41.84; | H, 4.68; | S, 7.60; | N, 26.55 |
| Found: | C, 41.93; | H, 4.43; | S, 7.48; | N, 26.34 |

Example 9

2-Amino-6-n-propoxypurine-9-β-D-2′,3′-dideoxyribofuranoside

2-Amino-6-n-propoxypurine (prepared by nucleophilic displacement of the chlorine group on 2-amino-6-chloropurine (Aldrich Chemical Co., Milwaukee WI) by the alkoxy anion formed between sodium hydride and propanol) (0.21g) and 3′-deoxythymidine (0.29g) (Horwitz J.P. et al , J.Org . Chem . 31 , 205 (1966)) were combined in 100ml potassium phosphate, pH 6.8, with 0.04% potassium azide. Purified thymidine phosphorylase (1200 I.U.) and purine nucleoside phosphorylase (8400 I.U.) (Krenitsky T.A., et al ., Biochemistry , 20 , 3615, 1981 and US Patent 4,381,444) were added and the suspension was stirred at 35°C.

After 48 hours the reaction was filtered, and the filtrate was chromatographed on a column containing AG-1 X2 hydroxide resin (2 X 5cm). The product was eluted with 90% methanol/water (v/v). The solvent was removed under vacuum, and the residue was dissolved in methanol. 10mls of dry silica gel were added, and the methanol was removed under vacuum. The dried silica gel was applied to a silica gel column (2.5 X 30cm) equilibrated in chloroform/methanol (9:1, v/v). This was also the eluting solvent. Fractions containing only product were combined and the solvent was removed under vacuum. The residual silica gel was removed, and the product was dried as described in Example 8. This yielded 0.132g of 2-amino-6-n-propoxypurine-9-β-D-2′,3′-dideoxyribofuranoside that analyzed as a 0.2 hydrate (mp = 70°C).

| Anal. Calcd. for $C_{13}H_{19}N_5O_3$ 0.2 $H_2O$: | | | |
|---|---|---|---|
| Calcd.: | C, 52.91; | H, 6.56; | N, 23.73 |
| Found: | C, 52.52; | H, 6.62; | N, 23.49 |

Example 10

6-Ethylthiopurine-9-β-D-2′,3′-dideoxyribofuranoside

6-Ethylthiopurine (5.5 mmoles, 1g) obtained from Sigma Chemical Co., St. Louis MO and 3′-deoxythymidine (4.47 mmoles) (Horwitz, J.P. et al ., J . Org . Chem ., 31 , 205 (1966)) were suspended in 50 ml of a 15 mM potassium phosphate solution with a pH of 7.2. Purified thymidine phosphorylase (7890 I. U.) and purine nucleoside phosphorylase (1980 I. U.) (Krenitsky T.A., et al ., Biochemistry , 20 3615, 1981 and US Patent 4,381,344) were added and the suspension stirred at 35°C. After 144 hours the reaction was filtered and the filtrate stored at -20°C. After thawing, the filtrate was adjusted to pH 10.7 with ammonium hydroxide and chromatographed on a column containing Dowex-1-formate resin (2.5 x 8 cm). This column was eluted with 30%n-propanol/water (v/v). Fractions containing product were combined and the solvent removed under vacuum. The residue was dissolved in 30% n-propanol/water (v/v) and chromatographed on a column containing BioRad P-2 (5 x 90 cm). The product was eluted from the column with 30% n-propanol/water (v/v). Product containing fractions were combined and the solvent removed under vacuum yielding 0.427g of 6-ethylthiopurine-9-β-D-2′.3′-dideoxyribofuranoside that analyzed as a 0.5 hydrate.

| Anal. Calcd. for $C_{12}H_{16}SN_4O_2 0.5 H_2O$: | | | | |
|---|---|---|---|---|
| Calcd.: | C, 49.81; | H, 5.92; | N, 19.36; | s, 11.44 |
| Found: | C, 49.63; | H, 5.95; | N, 19.28; | s, 11.06 |

NMR data: δ 8.71 (s, 1H, $H_8$), 8.67 (s, 1H, $H_2$), 6.33 (t,1H, $H_{1'}$), 4.1 (m, 2H, OH, $H_{4'}$), 3.4-3.6 (m,2H, 5′$CH_2$,) 1.8-2.4 (m, 4H, 2′ and 3′$CH_2$), 1.5′ (t, 3H, $CH_3$).

Example 11

2-Amino-6-Benzylthiopurine-9-β-D-2′,3′-dideoxyribofuranoside

2-Amino-6-benzylthiopurine (1.9 mmoles, 0.5 g) obtained from Sigma Chemical Co., St. Louis, MO and 3′-deoxythymidine (2.0 mmoles, 0.543 g) (Horwitz, J.P. et al ., J . Org . Chem . 31 205 (1966)) were dissolved in 20 ml of 10 mM potassium phosphate buffer, pH 7, containing 0.04% potassium azide. Purified thymidine phosphorylase (2,000 I.U.) and purine nucleoside phosphorylase (2,900 I.U.) (Krenitsky T.A., et al ., Biochemistry , 20 , 3615, 1981 and US Patent 4,381,344) were added and the suspension was stirred at 35°C. After three days, 80 ml of 10 mM potassium phosphate buffer, pH 7, were added. One day later the reaction was filtered. The cake was dissolved in 90% methanol/water (v/v), filtered, and the filtrate was chromatographed on a 2.5 x 10 cm column containing Dowex-1-hydroxide. The product was eluted from the column with 90% methanol/water (v/v). Product containing fractions were combined and after lyophilization yielded 0.086g of 2-amino-6-benzylthiopurine-9-β-D-2′,3′-dideoxyribofuranoside.
Anal. Calcd. for $C_{17}H_{19}SH_5O_2$: C, 57.13; H, 5.36; N, 19.59; S 8.97
Found: C, 57.02; H, 5.39; N, 19.51; S, 8.89
NMR data: δ 8.18 (s, 1 H, $H_8$), 7.3 (m, 5 H, ϕ), 6.6 (s, 2H, $NH_2$) 6.08 (dd, 1 $H_{1'}$), 4.93 (b, 1 H, 5′ OH), 4.45 (b, 2H, $CH_2$), 4.08 (m,1H, $H_{4'}$), 3.43-3.65 (m, 2 H, 5′ $CH_2$), 2.35 (m, 2 H, 2′ $CH_2$), 2.0 (m, 2 H, 3′ $CH_2$).

Example 12

6-Ethoxypurine-9-β-D-2′,3′-dideoxyribofuranoside

6-Ethoxypurine (3.0 mmoles, 0.5g: Sigma Chemicals Co., St. Louis MO) and 3′-deoxythymidine (3.3 mmoles, 0.75g) (Horwitz, J.P., et al ., J. Org. Chem. 31, 205, (1966)) were suspended in 25ml of 10 mM potassium phosphate buffer pH 6.8 and containing 0.04% potassium azide. Purified thymidine phosphorylase (800 I. U.) and purine nucleoside phosphorylase (1,200 I. U.) (Krenitsky T.A. et al ., Biochemistry , 20 , 3615, 1981 and US Patent 4,381,344) were added and the suspension was stirred at 35°C. After 24 hours, 85ml of 10 mM potassium phosphate buffer pH 6.8, were added and the reaction stirred for an additional five days at 35°C. The reaction precipitate was removed by filtration and the filtrate chromatographed on a 2.5 x 10 cm column containing Dowex-1-hydroxide. The product was eluted with 90% methanol/water (v/v) and the product containing fractions combined. After removing the solvent by vacuum, the material was dissolved in 30% n-propanol/water (v/v) and chromatographed on a 5 x 90 cm column containing BioRad P-2 resin. Product containing fractions were pooled and after lyophilization yielded 0.225g of 6-ethoxypurine-9-β-D-2′,3′-dideoxyribofuranoside that analyzed as a 0.15 hydrate.
Anal. Calcd. for $C_{13}H_{17}N_5O_2$ $0.15H_2O$: C, 53.98; H, 6.15; N, 20.98
Found: C, 54.05; H, 6.15; N, 20.88
NMR data: δ 8.6 (s, 1 H, $H_8$), 8.5 (s, 1H, $H_2$), 6.3 (dd, 1 H, $H_{1'}$), 4.97 (t, 1 H, 5′ OH), 4.6 (m, 2 H, $-CH_2-$), 4.1 (m 1 H, $H_{4'}$), 3,53 (m, 2 H, 5′ $CH_2$), 2.41 (m, 2 H, 2′ $CH_2$), 2.03 (m, 2 H, 3′ $CH_2$), 1.4 (t, 3 H, $CH_3$).

Example 13

6-Dimethylaminopurine-9-β-D-2′3′-dideoxyribofuranoside

6-Dimethylaminopurine (6.13 mmoles, 1g, Sigma Chemical Co., St. Louis, MO) and 3′-deoxythymidine (4.44 mmoles, 1g) (Horwitz, J. P. et al ., J. Org . Chem., 31 , 205 (1966) were suspended in 50ml of a 10 mM potassium phosphate solution pH 7.0 and containing 0.04% potassium azide. Purified thymidine phosphorylase (2000 I. U) and purine nucleoside phosphorylase (3000 I. U.) (Krenitsky T.A. et al ., Biochemistry , 20 , 3615, (1981) and US Patent 4,381,344) were added and the suspension stirred at 35°C. After 120 hours the reaction was filtered and the filtrate chromatographed on a column containing Dowex-1-hydroxide resin (2.5 x 8 cm) with 90% methanol and water (v/v) as the eluent. Fractions containing product were combined and the solvent removed under vacuum. The residue was dissolved in 25 ml 30% n-propanol/water (v/v) and chromatographed on a column containing BioRad P-2 (5 x 90cm). The product was eluted with 30% n-propanol/water (v/v). Product containing fractions were combined and the solvent removed under vacuum. The residue was dissolved in 30ml de-ionized water and chromatographed on a column containing Sephadex G-10 resin (5 x 90cm). The eluent was water. Appropriate fractions were combined and after lyophilization yielded 6-dimethylaminopurine-9-β-D-2′,3′-dideoxyribofuranoside that analyzed as a 0.3 hydrate and (mp = 162°C).

Anal. Calcd. for $C_{12}H_{17}N_5O_2 0.3H_2O$ C, 53.64; H, 6.60; N 26.06
Found: C, 53.63; H, 6.63; N, 25.8

Example 14

6-Hydroxyethylaminopurine-9-β-D-2′,3′-dideoxyribofuranoside

6-Hydroxyethylaminopurine (2.8 mmoles, 0. 5g, Sigma Chemical Co. St. Louis, MO) and 3′-deoxythymidine (3.30 mmoles, 0.76 g) (Horwitz J.P. et al , J.Org . Chem ., 31 205, (1966)) were suspended in 75 ml of a 10 mM potassium buffer, pH of 6.8 and containing 0.04% potassium azide. Purified thymidine phosphorylase (400 I.U.) and purine nucleoside phosphorylase (700 I.U.) (Krenitsky T.A., et al , Biochemistry , 20 3615, 1981 and U.S. Patent 4,381,344) were added and the suspension was stirred at 35°C. After 8 days, 600 I.U. thymidine phosphorylase and 1050 I.U. purine nucleoside phosphorylase were added. After an additional day, the reaction was filtered and the filtrate was applied to a 2.5 x 10cm column contained Dowex-1-hydroxide. The product was eluted with methanol. Product containing fractions were combined and evaporated under vacuum. The residue was then applied and eluted from a 2.5 x 50 cm silica gel column under pressure with a mixture of (8:2) chloroform: methanol. Product containing fractions were combined and after lyophilization yielded 6-hydroxyethylaminopurine-9-β-D-2′,3′-dideoxyribofuranoside that analyzed as a 0.65 hydrate and (mp = 153°C).

| Anal Calcd. for $C_{12}H_{17}N_5O_3 0.65H_2O$: | | | |
|---|---|---|---|
| Calcd: | C, 49.53; | H, 6.34; | N, 24.07 |
| Found: | C, 49.85; | H, 6.07; | N, 23.70. |

Example 15

6-Cyclopropylaminopurine-9-β-D-2′,3′-dideoxyribofuranoside

6-Cyclopropylaminopurine (prepared from 6-chloropurine (Sigma Chemicals, St. Louis MO) and cyclopropylamine) (2.86 mmoles, 0.5 g) and 3′-deoxythymidine (4.30 mmoles, 1 g) (Horwitz J.P. et al J.Org.Chem ., 31 , 205 (1966)) were dissolved in 10 ml of a 1:1 dimethylsulfoxide: N′,N′-dimethylformamide mixture and further diluted with 30 ml of a 10 mM potassium phosphate buffer pH 6.8 and containing 0.04% potassium azide. Purified thymidine phosphorylase (10,000 I.U.) and purine nucleoside phosphorylase (20,000 I.U.) (Krenitsky T.A., et al , Biochemistry , 20 , 3615, 1981 and US Patent 4,381,344) absorbed onto 10 ml of DEAE resin (Whatman) were added and the suspension was stirred at 35°C. After 8 hours the reaction was filtered and the filtrate was applied to a series of coupled columns. The initial column contained Dowex-1-hydroxide (2.5 x 10 cm) while the second column was filled with Amberlite XAD-2 resin

(2.5 x 20 cm). After sample application, the columns were washed with a large volume of water and the product was eluted with methanol. Product containing fractions were combined and after lyophilization yielded 0.54 g of 6-cyclopropylaminopurine-9-β-D-2′,3′-dideoxyribofuranoside that analyzed as a 0.55 hydrate and (mp = 63-65°C).

| Anal. Calcd. for $C_{13}H_{17}N_5O_2 0.55\ H_2O$: | | | |
|---|---|---|---|
| Calcd: | C, 54,75; | H, 6.40; | N, 24.55 |
| Found: | C, 54.67; | H, 6.43; | N, 24.57 |

Example 16

6-Cyclopentylaminopurine-β-D-2′,3′-dideoxyribofuranoside

6-Cyclopentylaminopurine (prepared from 6-chloropurine (Sigma Chemicals, St. Louis MO) and cyclopentylamine) (2.49 mmoles, 0.506 g) was dissolved in 5 ml N,N-dimethylformamide and 5 ml dimethylsulfoxide. 3′-deoxythymidine (3.94 mmoles, 0.894 g) (Horwitz, J.P. et al J.Org.Chem ., 31 , 205 (1966))was added along with 30 ml of 10 mM potassium phosphate buffer, pH 6.8 and 0.04% potassium azide. The pH was adjusted to 6.8 with acetic acid. Purified thymidine phosphorylase (10,000 I.U.) and purine nucleoside phosphorylase (20,000 I.U.) (Krenitsky T.A., et al , Biochemistry , 20 , 3615, 1981 and US Patent 4,381,344) bound to DEAE-cellulose (Whatman) was added to the reaction mixture. The suspension was stirred at 35°C for 8 hours, filtered, and the filtrate stored overnight at -20°C. Upon thawing, the filtrate was applied to a 2.5 x 10 cm column containing Dowex-1-hydroxide resin. The product was eluted with water. Product containing fractions were combined and chromatographed on a column containing XAD-2 resin (2.5 x 20 cm). This product was eluted with 350 ml of water followed by methanol. Product containing fractions were combined and the methanol removed under vacuum. The residue was dissolved in water and after lyophilization, yielded 0.459 g of 6-cyclopentylaminopurine-β-D- 2′,3′-dideoxyribofuranoside that analyzed as a 0.05 hydrate and (mp = 88°C).

| Anal. Calcd. for $C_{15}H_{21}N_5O_2 0.05\ H_2O$ | | | |
|---|---|---|---|
| Calcd; | C, 59.21 | H, 6.99; | N, 23.02 |
| Found; | C, 59.24; | H, 7.05; | N,22.95 |

Example 17

2-Amino-6-methoxypurine-9-β-D-2′,3′-dideoxyribofuranoside

2-Amino-6-methoxypurine (3.0 mmoles, 0.5 g, prepared from 2-amino-6-chloropurine (Aldrich Chemical Co., Milwaukee WI) and methanol) and 3′-deoxythymidine (4.50 mmoles, 1g) (Horwitz J.P. et al , J.Org.Chem ., 31 , 205 (1966)) were dissolved in 10 ml of a 1:1 dimethylsulfoxide:N′,N′-dimethylformamide mixture and further diluted with 30 ml of a 10 mM potassium phosphate buffer with a pH of 6.8 and containing 0.04% potassium azide. Purified thymidine phosphorylase (10,000 I.U) and purine nucleoside phosphorylase (20,000 I.U.) (Krenitsky, et al ., Biochemistry , 20 , 3615, 1981 and US Patent 4,381,344) adsorbed onto 10 ml of DEAE resin were added and the suspension was stirred at 35°C. After 8 hours the reaction was filtered and the filtrate was applied to a 2.5 x 10 cm column containing Dowex-1-hydroxide. Fractions containing product were pooled and reduced to a volume of 70 mls. This sample was applied to a 2.5 x 20 cm column filled with Amberlite XAD-2 resin. The column was washed with a large volume of water and the product was eluted with methanol. Product containing fractions were combined and after lyophilization yielded 2-amino-6-methoxypurine-9-β-D-2′,3′-dideoxyribofuranoside.
Anal. Calcd. for $C_{11}H_{15}N_5O_3$ : C, 49.81; H, 5.70; N, 26.40
Found: C, 49.70; H, 5.72; N, 26.34.

Example 18

6-n-Propoxypurine-9-β-D-2′,3′-dideoxyribofuranoside

6-n-Propoxypurine (0.5g, 2.8 mmoles, Sigma Chemicals, St. Louis, MO) and 3′-deoxythymidine (0.96g, 4.2 mmoles)(Horwitz, J.P., et al J.Org.Chem ., 31 , 205 (1996)) were dissolved in 5ml dimethyl sulfoxide and 5ml N,N dimethylformamide. 30ml of 10mm potassium phosphate buffer, pH 6.8, containing 0.04% potassium azide and purified purine nucleoside phosphorylase (20,000 I.U.) and thymidine phosphorylase (10,000 I.U.) (Krenitsky, T.A et al ., Biochemistry , 20 , 3615, 1981 and US Patent 4,381,344) absorbed onto 10ml of DEAE-cellulose resin were added and the reaction was stirred at 35°C for 7 hours. The resin was removed by centrifugation and the supernatant applied to a column of AG1-X2 (OH form), 2.5 x 10cm, coupled to a column of XAD-2, 2.5 x 20cm. The columns were washed with 500ml of water and the product was eluted with methanol. The product was flash chromatographed on a silica gel column, 3 x 50 cm, with chloroform : methanol (9:1 v/v). Lyophilization afforded 0.554g of 6-n-propoxypurine-9-β-D-2′,3′-dideoxyribofuranoside that analyzed as a 0.3 hydrate.

| Analysis Calculated for $C_{13}H_{18}N_4O_3 0.3H_2O$ | | | |
|---|---|---|---|
| Calculated : | C, 55.04; | H, 6.61; | N, 19.75 |
| Found : | C, 55.05; | H, 6.61; | N, 19.72 |

Example 19

6-n-Butoxypurine-9-β-D-2′,3′-dideoxyribofuranoside

6-n-Butoxypurine (0.5g, 2.6 mmoles, Sigma Chemicals, St. Louis, MO) and 3′-deoxythymidine (0.70g, 3.1 mmoles) (Horwitz J.P. et al , J.Org.Chem., 31, 205 (1966)) were suspended in 100ml of 10mM potassium phosphate buffer, pH 6.8, containing 0.04% potassium azide. Purified purine nucleoside phosphorylase (3,500 I.U.) and thymidine phosphorylase (800 I.U.) (Krenitsky, T.A., et al ., Biochemistry , 20 , 3615, 1981 and US Patent 4,381,344) were added and the solution was stirred at 32°C. After 7 days the reaction was filtered and the filtrate applied to a column containing AG1-X2 (OH- form), 2.5 x 10cm. Product was eluted with 90% aqueous methanol. Solvent was removed in vacuo from the product and the residue was flash chromatographed on a silica gel column, 2.5 x 80 cm, with chloroform : methanol (8:2, v/v). The product was dissolved in water and applied to a column containing XAD-2, 2.5 x 20cm. The column was washed with 500ml of water and then developed with methanol. Lyophilization yielded 0. 276g of 6-n-butoxypurine-9-β-D-2′,3′-dideoxyribofuranoside (mp 55°C).

| Analysis Calculated for $C_{14}H_{20}N_4O_3$ | | | |
|---|---|---|---|
| Calculated : | C, 57.52; | H, 6.90; | N, 19.17 |
| Found : | C, 57.86; | H, 7.29; | N, 18.83 |

Example 20

6-Cyclopropylmethoxypurine-9-β-D-2′,3′-dideoxyribofuranoside

6-Cyclopropylmethoxypurine was prepared by nucleophilic displacement of the chlorine group on 6-chloropurine (Sigma Chemical Co., St. Louis MO) by the alkoxy anion formed between sodium hydride and cyclopropylmethyl alcohol.

6-Cyclopropylmethoxypurine (0.505g, 26.5 mmoles) and 2′,3′-dideoxythymidine (0.908g, 40.1 mmoles) (Horwitz et al , J.Org.Chem ., 31 , 205 (1966)) were reacted and chromatographed on AG1-X2 (OH form) and XAD-2 as described in Example 18. Product containing fractions were flash chromatographed on a silica gel column, 3 x 50 cm, with acetonitrile : water (98:2, v/v). Lyophilization yielded 0.496g of 6-cyclopropylmethoxypurine-9-β-D-2′,3′-dideoxyribofuranoside.

| Analysis Calculated for $C_{14}H_{18}N_4O_3$ | | | |
|---|---|---|---|
| Calculated : | C, 57.92; | H, 6.25; | N, 19.30 |
| Found : | C, 57.99; | H, 6.28; | N, 19.27 |

Example 21

6-Cyclopentyloxypurine-9-β-D-2′,3′-dideoxyribofuranoside

6-Cyclopentyloxypurine was prepared by nucleophilic displacement of the chlorine group on 6-chloropurine (Sigma Chemical Co., St. Louis, 31, 205 (1966)) by the alkoxy anion formed between sodium hydride and cyclopentanol.

6-Cyclopentyloxypurine (0.506g, 2.48 mmoles) and 3′-deoxythymidine (0.856g, 3.78 mmoles) (Horwitz J.P., et al , J.Org.Chem , 31 , 205 (1966)) were reacted and chromatographed on AG1-X2 (OH form) and XAD-2 as described in Example 18. Solvent was removed in vacuo from product fractions and the residue was flash chromatographed on a silica gel column, 3 x 50cm, with chloroform : methanol (95.5, v/v). Lyophilization yielded 0.385g of 6-cyclopentyloxypurine-9-β-D-2′,3′-dideoxyribofuranoside that analyzed as a 0.15 hydrate.

| Analysis Calculated for $C_{15}H_{20}N_4O_3$ 0.15$H_2O$ | | | |
|---|---|---|---|
| Calculated : | C, 58.68; | H, 6.66; | N, 18.25 |
| Found : | C, 58.61; | H, 6.53; | N, 18.25 |

Example 22

6-Cyclohexyloxypurine-9-β-D-2′,3′-dideoxyribofuranoside

6-Cyclohexyloxypurine was prepared by nucleophilic displacement of the chlorine group on 6-chloropurine (Sigma Chemical Co., St. Louis MO) by the alkoxy anion formed between sodium hydride and cyclohexanol.

6-Cyclohexyloxypurine (0.50g, 2.29 mmoles) and 3′-deoxythymidine (0.776g, 3.42 mmoles) (Horwitz J.P. et al J.Org.Chem ., 31 , 205 (1966)) chromatographed on AG1-X2 (OH form) and XAD-2 as described in Example 18 with the exception that 10ml glyme in addition to the 5ml dimethyl sulfoxide and 5ml N,N-dimethylformamide, and a total of 70 ml of 10mM potassium phosphate buffer, pH 6.8, containing 0.04% potassium azide were used. Lyophilization yielded 0.102g of 6-cyclohexyloxypurine-9-β-D-2′,3′-dideoxyribofuranoside (mp 105°C) that analyzed as a 0.2 hydrate.

| Analysis Calculated for $C_{16}H_{22}N_4O_3$ 0.2$H_2O$ | | | |
|---|---|---|---|
| Calculated :<br>Found : | C, 59.69;<br>C, 59.69; | H, 7.01;<br>H, 6.93; | N, 17.40<br>N, 17.27 |

Example 23

6-Cyclobutylaminopurine-9-β-D-2′,3′-dideoxyribofuranoside

6-Cyclobutylaminopurine was prepared by nucleophilic displacement of the chlorine group on 6-chloropurine (Sigma Chemical Co., St. Louis MO) by the amino group on cyclobutylamine.

6-Cyclobutylaminopurine (0.510g, 2.62 mmoles) and 3′-deoxythymidine (0.896g, 3.96 mmoles) (Horwitz J.P. et al J.Org.Chem ., 31 , 205 (1966)) were reacted and chromatographed on AG1-X2 (OH form) and XAD-2 as described in Example 18. Solvent was removed from product containing fractions and the residue was flash chromatographed on a silica gel column, 3 x 50cm, with chloroform : methanol (9:1, v/v). Lyophilization yielded 0. 524g of 6-cyclobutylaminopurine-9-β-D-2′,3′-dideoxyribofuranoside (mp 96-98°C).

| Analysis Calculated for $C_{14}H_{19}N_5O_2$ | | | |
|---|---|---|---|
| Calculated :<br>Found : | C, 58.12;<br>C, 58.19; | H, 6.62;<br>H, 6.65; | N, 24.20<br>N, 24.16 |

Example 24

6-Diethylaminopurine-9-β-D-2′,3′-dideoxyribofuranoside

6-Diethylaminopurine was prepared by nucleophilic displacement of the chlorine group on 6-chloropurine (Sigma Chemical Co., St. Louis MO) by the amino group on diethylamine.

6-Diethylaminopurine (0.246g 1.28 mmoles) and 3′-deoxythymidine (0.463g, 2.04 mmoles) (Horwitz J.P. et al J.Org.Chem ., 31 , 205 (1966)) were reacted and chromatographed on AG1-X2 (OH form) and XAD-2 as described in Example 18. Solvent was removed in vacuo from product containing fractions and the residue was flash chromatographed on a silica gel column, 5 x 20cm with chloroform : methanol (9:1, v/v). Solvent was removed in vacuo from product containing fractions and the residue was flash chromatographed on a second silica gel column, 2.5 x 50cm, with ethyl acetate. The product gum was transferred in acetone to a vial and lyophilization yielded 0.098g of 6- diethylaminopurine-9-β-D-2′,3′-dideoxyribofuranoside that analyzed for 0.25 water and 0.20 acetone.

| Analysis Calculated for $C_{14}H_{21}N_5O_2$ 0.2$C_3H_6O$ 0.25$H_2O$ | | | |
|---|---|---|---|
| Calculated :<br>Found : | C, 57.03;<br>C, 57.02; | H, 7.44;<br>H, 7.39; | N, 22.78<br>N, 22.72 |

Example 25

6-Pyrrolidinopurine-9-β-D-2′,3′-dideoxyribofuranoside

6-Pyrrolidinopurine was prepared by nucleophilic displacement of the chlorine group on 6-chloropurine by the amino group on pyrrolidine.

6-Pyrrolidinopurine (0.500g, 2.64 mmoles) and 3-deoxythymidine (0.901g, 3.98 mmoles) (Horwitz J.P. et al J.Org.Chem ., 31 , 205 (1966)) were dissolved in 5 ml dimethyl sulfoxide and 5 ml N,N-dimethylformamide. Thirty ml of 10 mM potassium phosphate buffer, pH 6.8 containing 0.04% potassium azide and purified purine nucleoside phosphorylase (20,000 I.U) and thymidine phosphorylase (10,000 I.U) (Krenitsky, T.A. et al ., Biochemistry , 20 , 3615, 1981 and US Patent 4,381,344) adsorbed onto 10 ml of DEAE-cellulose resin were added and the reaction was stirred at 35°C for 7 hours. The resin was removed by centrifugation and the supernatant applied to a column of AG1-X2 (OH-form), 2.5 x 10 cm, coupled to a column of XAD-2, 2.5 x 20 cm. The columns were washed with 500 ml of water and the product was eluted with methanol. Lyophilization yielded 0.385g of 6-pyrrolidinopurine-9-β-D-2′,3′-dideoxyribofuranoside that analyzed as a 0.05 hydrate (mp 158-159°C).

| Analysis Calculated for $C_{14}H_{19}N_5O_2$ 0.05$H_2O$ | | | |
|---|---|---|---|
| Calculated : | C, 57.94; | H, 6.63; | N, 24.13 |
| Found : | C, 57.92; | H, 6.67; | N, 24.11 |

Example 26

6-Morpholinopurine-9-β-D-2′,3′-dideoxyribofuranoside

6-Morpholinopurine was prepared by nucleophilic displacement of the chlorine group on 6-chloropurine (Sigma Chemical Co., St. Louis MO) by the amino group on morpholine.

6-Morpholinopurine (0.501g, 2.44 mmoles) and 3′-deoxythymidine (0.842g, 3.72 mmoles)(Horwitz J.P. et al J.Org.Chem ., 31 , 205, (1966)) were reacted and chromatographed on AG1-X2 (OH form) and XAD-2 as described in Example 18. Lyophilization yielded 0.292g of 6-morpholinopurine-9-β-D-2′,3′-dideoxyribofuranoside that analyzed as a 0.2 hydrate (mp 97°C).

| Analysis Calculated for $C_{14}H_{19}N_5O_3$ 0.20$H_2O$ | | | |
|---|---|---|---|
| Calculated : | C, 54.43; | H, 6.33; | N, 22.67 |
| Found : | C, 54.48; | H, 6.28; | N, 22.51 |

Example 27

6-y,y-Dimethylallylaminopurine-9-β-D-2′,3′-dideoxyribofuranoside

6-γ,γ-Dimethylallylaminopurine (0.500g, 2.46 mmoles, Sigma Chemicals, St. Louis, MO) and 3′-deoxythymidine (0.752g, 3.32 mmoles) (Horwitz J.P. et al , J.Org.Chem ., 31 , 205 (1966)) were reacted and chromatographed on AG1-X2 (OH form) as described in Example 18. Solvent was removed in vacuo from product containing fractions and the residue was flash chromatographed on a silica gel column, 3 x 50 cm, with chloroform : methanol (95:5, v/v). Product containing fractions were then applied to an XAD-2 column, 2.5 x 20 cm, and eluted with methanol. The product gum was transferred in acetone to a vial and lyophilization yielded 0.445g of 6-γ-γ-dimethylallylaminopurine-9-β-D-2′,3′-dideoxyribofuranoside that analyzed for 0.45 water and 0.20 acetone.

| Analysis Calculated for $C_{15}H_{21}N_5O_2$ 0.45$H_2O$ 0.2$C_3H_6O$ | | | |
|---|---|---|---|
| Calculated : | C, 57.99; | H, 7.21; | N, 21.68 |
| Found : | C, 57.77; | H, 6.91; | N, 21.41 |

Example 28

6-Furfurylaminopurine-9-β-D-2′,3′-dideoxyribofuranoside

6-Furfurylaminopurine (0.502g, 2.33 mmoles, Sigma Chemicals, St. Louis, MO) and 3′-deoxythymidine (0.754g, 3.33 mmoles) (Horwitz J.P. et al J.Org.Chem., 31, 205 (1966)) were reacted and chromatographed on AG1-X2 (OH form) and XAD-2 as described in Example 18. Solvent was removed in vacuo from product containing fractions and the residue was flash chromatographed on a silica gel column, 5 x 50 cm, with chloroform : methanol (9:1, v/v). Lyophilization yielded 0.303g of 6-furfurylaminopurine-9-β-D-2′,3′-dideoxyribofuranoside that analysed as a 0.2 hydrate.

| Analysis Calculated for $C_{15}H_{17}N_5O_3$ 0.2$H_2O$ | | | |
|---|---|---|---|
| Calculated : | C, 56.49; | H, 5.50; | N, 21.96 |
| Found : | C, 56.50; | H, 5.53; | N, 21.97 |

Example 29

6-Benzylmercaptopurine-9-β-D-2′,3′-dideoxyribofuranoside

6-Benzylmercaptopurine (0.501g, 2.07 mmoles, Sigma Chemicals, St. Louis, MO) and 3′deoxythymidine (0.704g, 3.11 mmoles) (Horwitz J.P. et al J.Org.Chem., 31, 205, (1966)) were reacted and chromatographed on AG1-X2 (OH form) as described in Example 18 except that 10ml glyme was used to dissolve the purine base. Solvent was removed in vacuo from product containing fractions and the residue was flash chromatographed on a silica gel column, 3 x 50 cm, with chloroform : methanol (95:5, v/v). The product was transferred in ethanol to a vial and lyophilization yielded 0.304g of 6-benzylmercaptopurine-9-β-D-2′,3′-dideoxyribofuranoside that analyzed for 0.05 water and 0.05 ethanol (mp 81-83°C).

| Analysis Calculated for $C_{17}H_{18}N_4O_2S$ 0.05$H_2O$ 0.05$C_2H_6O$ | | | | |
|---|---|---|---|---|
| Calculated : | C, 59.43; | H, 5.37; | N, 16.21; | S, 9.28 |
| Found : | C, 59.49; | H, 5.38; | N, 16.32; | S, 9.30 |

Example 30

6-Anilinopurine-9-β-D-2′,3′-dideoxyribofuranoside

6-Anilinopurine (0.500g, 2.37 mmoles, Sigma Chemicals, St. Louis, MO) and 3′-deoxythymidine (0.752g, 3.32 mmoles) (Horwitz J.P. et al J.Org.Chem., 31, 205 (1966)) were reacted and chromatographed on

AG1-X2 (OH form) as described in Example 15. Solvent was removed in vacuo from product containing fractions and the residue was flash chromatographed on a silica gel column, 2.5 x 50 cm, with chloroform : methanol (95:5, v/v). Lyophilization yielded 0.470g of 6-anilinopurine-9-β-D-2′,3′-dideoxy- ribofuranoside that analyzed as a 0.05 hydrate (mp 170-172°C).

| Analysis Calculated for $C_{16}H_{17}N_5O_2$ 0.05$H_2O$ | | | |
|---|---|---|---|
| Calculated :<br>Found : | C, 61.55;<br>C, 61.57; | H, 5.52;<br>H, 5.55; | N, 22.43<br>N, 22.43 |

Example 31

2-Amino-6-ethoxypurine-9-β-D-2′,3′-dideoxyribofuranoside

2-Amino-6-ethoxypurine (0.5g, 2.8 mmoles prepared by nucleophilic displacement of the chlorine group on 2-amino-6-chloropurine, (Aldrich Chemical Co. , Milwaukee WI) by the alkoxy anion formed between sodium hydride and ethanol) and 3′-deoxythymidine (0.950g, 4.19 mmoles) (Horwitz J.P. et al J.Org.Chem ., 31 , 205 (1966)) were reacted and chromatographed on AG1-X2 (OH form) and XAD-2 as described in Example 18. Solvent was removed in vacuo from product containing fractions and the residue was flash chromatographed on a silica gel column, 5 x 20 cm, with chloroform : methanol (9:1, v/v). Lyophilization yielded 0.443g of 2-amino- 6-ethoxypurine-9-β-D-2′,3′-dideoxyribofuranoside that analyzed as a 0.3 hydrate (mp 150°C, partial melt at 65°C).

| Analysis Calculated for $C_{12}H_{17}N_5O_3$ 0.3$H_2O$ | | | |
|---|---|---|---|
| Calculated :<br>Found : | C, 50.63;<br>C, 50.77; | H, 6.23;<br>H, 6.21; | N, 24.60<br>N, 24.63 |

Example 32

2,6,8-Triaminopurine-9-β-D-2′,3′-dideoxyribofuranoside

2,6,8-Triaminopurine (0.500g, 3.0 mmoles) (Davies, R., et al ., Biochim . Biophys , Acta ., 564(3), 448, 1979) and 3′-dideoxythymidine (1.02g, 4.50 mmoles) (Horwitz J.P. et al J.Org.Chem . 31 , 205 (1966)) were reacted and chromatographed on AG1-X2 (OH form) and XAD-2 as described in Example 18. Lyophilization yielded 0.148g of 2,6,8-triaminopurine-9-β-D-2′,3′-dideoxyribofuranoside that analyzed for 0.7 methanol (mp 154°C).

| Analysis calculated for $C_{10}H_{15}N_7O_2$ 0.7$CH_4O$ | | | |
|---|---|---|---|
| Calculated :<br>Found : | C, 44.76;<br>C, 44.51; | H, 6.24;<br>H, 5.95; | N, 34.08<br>N, 33.78 |

Example 33

2-Amino-6-benzylaminopurine-9-β-D-2′,3′-dideoxyribofuranoside

2-Amino-6-benzylaminopurine (0.2g, 0.8 mmoles prepared by nucleophilic displacement of the chlorine group on 2-amino-6-chloropurine (Aldrich Chemical Co. Milwaukee WI) by benzylamine) and 3′-deoxythymidine (0.282g, 1.2 mmoles) (Horwitz J.P. et al J.Org.Chem ., 31 , 205 (1966)) were reacted and chromatographed on AG1-X2 (OH form) and XAD-2 as described in Example 18 except smaller amounts of purine nucleoside phosphorylase (10,000 I.U.) and thymidine phosphorylase (5,000 I.U.) were used. Lyophilization yielded 0.182g of 2-amino-6-benzylaminopurine-9-β-D-2′,3′-dideoxyribofuranoside that analyzed for 0.60 methanol (mp 92-94°C).

| Analysis Calculated for $C_{17}H_{20}N_6O_2$ 0.60$CH_4O$ | | | |
|---|---|---|---|
| Calculated : | C, 58.78; | H, 6.28; | N, 23.37 |
| Found : | C, 58.60; | H, 6.06; | N, 23.48 |

Example 34

2-Amino-6-cyclopropylaminopurine-9-β-D-2,′3′-dideoxyribofuranoside

2-Amino-6-cyclopropylaminopurine (0.495g, 2.1 mmoles prepared by nucleophilic displacement of the chlorine group on 2-amino-6-chloropurine (Aldrich Chemical Co. Milwaukee WI) by cyclopropylamine) and 3′-deoxythymidine (0.73g, 3.2 mmoles) (Horwitz J.P. et al J.Org. Chem ., 31 , 205 (1966)) were reacted and chromatographed on AG1-X2 (OH form) and XAD-2 as described in Example 18. Lyophilization yielded 0.419g of 2-amino-6-cyclopropylaminopurine-9-β-D-2′-dideoxyribofuranoside that analyzed as a 0.3 hydrate (mp 82-84°C).

| Analysis Calculated for $C_{13}H_{18}N_6O_2$ 0.3$H_2O$ | | | |
|---|---|---|---|
| Calculated : | C, 52.80; | H, 6.34; | N, 28.42 |
| Found : | C, 52.83; | H, 6.35; | N, 28.44 |

Example 35

2-Amino-6-methylaminopurine-9-β-D-2′,3-dideoxyribofuranoside

2-Amino-6-methylaminopurine (0.5 g, 3.0 mmoles prepared by nucleophilic displacement of the chlorine group on 2-amino-6-chloropurine (Aldrich Chemical Co. Milwaukee WI) by methylamine) and 3′-deoxythymidine (0.893 g, 3.9 mmoles) (Horwitz, J.P. et al ., J. Org . Chem ., 31, 205 (1966)) were suspended in 100 ml of 10 mM potassium phosphate buffer, pH 6.8, containing 0.04% potassium azide. Purified purine nucleoside phosphorylase (2,880 I U) and thymidine phosphorylase (1.200 I.U.) (Krenitsky, T.A. et al ., Biochemistry , 20 , 3615, 1981 and US Patent 4,381,344) were added and the reaction was stirred at 33°C for 72 hours. The reaction was applied to a column of AG1-X2 (OH-form) 2.5 x 10 cm, and the product eluted with 90% aqueous methanol. Solvent was removed in vacuo and the residue was flash chromatographed on a silica gel column, 2.5 x 30 cm, with chloroform: methanol (97.3, v/v). Lyophilization yielded 0.3 g, of 2-amino-6-methylaminopurine-9-β-D-2′,3-dideoxyribofuranoside that analysed as a 0.4 hydrate (m.p. 95 °C partial melt at 75°C)

| Analysis Calculated for $C_{11}H_{16}N_6O_2$ 0.4$H_2O$ | | | |
|---|---|---|---|
| Calculated: | C, 48.66; | H, 6.24; | N, 30.95 |
| Found: | C, 48.57; | H, 6.27; | N, 30.77 |

Example 36

2-Amino-6-n-propoxypurine-9-β-D-2′,3′-dideoxyribofuranoside

2-Amino-6-n-propoxypurine (0.21 g, 1.1 mmoles prepared by nucleophilic displacement of the chlorine group on 2-amino-6-chlorpurine (Aldrich Chemical Co. Milwaukee WI) by the alkoxy anion formed between sodium hydride and n-propanol) and 3′-deoxythymidine (0.293 g, 1.3 mmoles) (Horwitz, J.P. et al , J . Org . Chem ., 31 , 205, (1966)) were suspended in 100 ml of 10 mM potassium phosphate buffer, pH 7.0 containing 0.04% potassium azide. Purified purine nucleoside phosphorylase (2,880 I.U) and thymidine phosphorylase (1200 IU) (Krenitsky, T.A, et . al ., Biochemistry , 20 , 3615, 1981 and US Patent 4,381,344) were added and the reaction was stirred at 33°C for 48 hours. The reaction was applied to a column of AG1-X2 (OH form) 2.5 x 5 cm, and eluted with 90% aqueous methanol. Solvent was removed in vacuo and the residue was flash chromatographed on a silica gel column 2.5 x 30 cm, with chloroform: methanol (9:1 v/v). Lyophilization yielded 0.132 g, of 2-amino-6-n-propoxypurine-9-β-D-2′,3′-dideoxyribofuranoside that analysed as a 0.2 hydrate (m.p. 70°C)

| Analysis Calculated for $C_{13}H_{19}N_5O_3$ 0.2$H_2O$ | | | |
|---|---|---|---|
| Calcuated: | C, 52.59; | H, 6.59; | N, 23.59 |
| Found: | C, 52.52; | H, 6.62; | N, 24.49 |

Example 37

6-Benzylaminopurine-9-β-D-2′,3′-dideoxyribofuranoside

6-Benzylaminopurine (1.0 g, 4.44 mmoles, Sigma Chemicals, St. Louis, MO) and 3′-deoxythymidine (1.0 g, 4.4 mmoles) (Horwitz, J.P. et al ., J . Org . Chem ., 31 , 205, (1966)) were suspended in 50 ml of 15 mM potassium phosphate buffer, pH 7.2. Purified purine nucleoside phosphorylase (2,000 I.U.) and thymidine phosphorylase (7,900 I.U.) (Krenitsky, T.A., et . al ., Biochemistry 20 , 3615, 1981 and US Patent 4,381,344) were added and the reaction was stirred at 25°C. After 1 hour, 6ml of diglyme were added and the reaction was stirred at 37°C for 6 days. The reaction filtrate was adjusted to pH 10.5 with ammonium hydroxide, applied to a column of AG1-X2 (formate form), 2 x 6 cm, and the product eluted with 30% aqueous propanol. The product was then chromatographed on a P-2 column, 2.5 x 90 cm, eluted with 30% aqueous propanol and lyophilization yielded 0.063 g of 6-benzylaminopurine-9-β-D-2′,3′-dideoxyribofuranoside that analysed as a 0.5 hydrate (m.p. 65°C).

| Analysis Calculated for $C_{17}H_{19}N_5O_2$ 0.5$H_2O$ | | | |
|---|---|---|---|
| Calculated: | C, 61.06; | H, 6.03; | N, 20.94 |
| Found: | C, 61.29; | H, 6.21; | N, 20.69 |

Example 38

6-iso-Propoxypurine-9-β-D-2′-3′-dideoxyribofuranoside

6-iso-Propoxypurine (0.5 g, 2.8 mmoles, Sigma Chemicals, St, Louis, MO) and 3′-deoxythmidine (0.95 g, 4.2 mmoles) (Horwitz, J.P. et al , J. Org. Chem., 31, 205, (1966)) were reacted and chromatographed on AG1-X2 (OH-form) and XAD-2 as described in Example 18. Solvent was removed in vacuo from product fractions and the residue was dissolved in 30% aqueous propanol. Chromatography on a G-10 column, 5 x 90 cm, developed with 30% aqueous propanol yielded a gum which was transferred in acetone to a lyophilisation flask. Lyophilisation yielded 0.313 g of 6-iso-propoxypurine-9-β-D-2′,3′-dideoxyribofuranoside that analysed for 0.2 water and 0.2 acetone (m.p. 75°C).

| Analysis Calculated for $C_{13}H_{18}N_4O_3$ $0.2C_3H_6O$ $0.2H_2O$ | | | |
|---|---|---|---|
| Calculated: | C, 55.65; | H, 6.73; | N, 19.09 |
| Found: | C, 55.65; | H, 6.59; | N, 19.12 |

Example 39

6-n-Propylaminopurine-9-β-D-2′,3′-dideoxyribofuranoside

6-n-Propylaminopurine (0.500 g, 2.81 mmoles, Sigma Chemicals, St. Louis, MO) and 3′-deoxythymidine (0.957 g, 4.26 mmoles) (Horwitz, J.P. et . al ., J.Org.Chem ., 31 , 205 (1966) were reacted and chromatographed on AG1-X2 (OH-form) and XAD-2 as described in Example 18 except than the 5ml dimethyl sulfoxide was replaced with an additional 5 ml N,N-dimethylformamide. Solvent was removed in vacuo from product containing fractions and the residue was flash chromatographed on a silica gel column, 3 x 50 cm, with chloroform: emthanol (9:1 v/v). Lyophilization yield 0.499 g of 6-n-propylaminopurine-9-β-D-2′,3′-dideoxyribofuranoside that analysed as a 0.7 hydrate.

| Analysis Calculated for $C_{13}H_{19}N_5O_2$ $0.7H_2O$ | | | |
|---|---|---|---|
| Calculated: | C, 53.85; | H, 7.09; | N. 24.15 |
| Found: | C, 53.93; | H, 7.08; | N, 24.18 |

Example 40

6-Cyclohexylaminopurine-9-β-D-2′,3′-dideoxyribofuranoside

6-Cyclohexylaminopurine was prepared by nucleophilic displacement of the chlorine group of 6-chloropurine by cyclohexylamine.

6-Cyclohexylaminopurine (1.0 g, 5 mmoles) and 3′-deoxythymidine (2.07 g, 9.1 mmoles) (Horwitz, J.P. et al ., J.Org.Chem . 31 , 205, (1966)) were dissolved in 25 ml 2-methoxyethyl ether and 500 ml of 10 mM potassium phosphate buffer, pH 7.2. Purified purine nucleoside phosphorylase (5,000 I.U) and thymidine phosphorylase (3850, I.U.) (Krenitsky, T.A. et . al ., Biochemistry , 20 , 3615, 1981 and US Patent 4,381,344) were added and the reaction was stirred at 37°C for 7 days. The reaction mixture was applied to a column of XAD-2 and washed extensively with water. Product was eluted with 90% aqueous methanol. UV absorbing fractions were pooled and applied to a column of AG1-X2 (OH-form), 2 x 12 cm, and the product

was eluted with 30% aqueous methanol. The product was further chromatographed on a P-2 column, 2.5 x 90 cm, and a G-10 column, 2.5 x 90 cm, and each column was eluted with 30% aqueous propanol. Lyophilisation yielded 0.093 g of 6-cyclohexylaminopurine-9-β-D-2′,3′-dideoxyribofuranoside (mp 70-72°C)

| Analysis Calculated for $C_{16}H_{23}N_5O_2$ | | | |
|---|---|---|---|
| Calculated:<br>Found: | C, 60.55;<br>C, 60.37; | H, 7.30;<br>H, 7.39; | N. 22.07<br>N, 21.94 |

Example 41

6-Methylaminopurine-9-β-D-2′,3′-dideoxyribofuranoside

6-Methylaminopurine (4.31 mmoles, 1g) obtained from Sigma Chemical Co., St. Louis, MO and 3′-deoxythymidine (4.40 mmoles, 1g)(Horwitz J.P. et al ; J.Org.Chem . 31 , 205(1966)) were suspended in 50ml of 10mM potassium phosphate buffer, pH 7, and 0.04% potassium azide. Purified thymidine phosphorylase (2,000 I.U.) and purine nucleoside phosphorylase (2,400 I.U.) (Krenitsky T.A., et al ., Biochemistry 20 , 3615, 1981 and US Patent 4,381,344) were added and the suspension was stirred at 35°C. After three days, the reaction was stored at -20°C. Upon thawing, the reaction was filtered and the filtrate applied to a 2.5 x 10 cm column containing Dowex-1-hydroxide. The product was eluted from the column with 90% methanol/water (v/v). Product containing fractions were combined and the solvent removed under vacuum. This material was chromatographed twice on a 5 x 90 cm column containing BioRad P-2 resin with 30% n-propanol/water (v/v). Product containing fractions were pooled, and after lyophilization yielded 0.391g of 6-methylaminopurine-9-β-D-2′,3′-dideoxyribofuranoside that analysed as a 0.1 hydrate.
Anal. Calcd. for $C_{11}H_{15}N_5O_2O$. $1H_2O$: C, 52.62; H, 6.10; N, 27.89
Found: C, 52.75; H, 6.16; N, 28.01
NMR data: δ 8.34 (s, 1 $H_8$), 8.12 (s, 1 H, $H_2$), 7.72 (b, 1 H, NH) 6.23 (dd, 1 H, $H_{1'}$), 5.06 (t, 1 H, 5′ OH), 4.10 (m, 1 H, $H_{4'}$,) 3.58-3.69 (M, 1 H 5′ $CH_2$), 3.45-3.55 (m, 1 H, 5′ $CH_2$), 2.95 (b, 3H, $CH_3$), 2.40 (m, 2H, 2′$CH_2$) and 2.07 (m, 2 H, 3′ $CH_2$).

Example 42

2-Amino-6-(cyclopropylmethylamino)purine-9-β-D-2′,3′-dideoxy ribofuranoside

6-(Cyclopropylmethylamino)purine (0.022 moles, 0.5 g) and 3′-deoxythymidine (0.0032 moles, 0.73 g) were suspended in 10 mL dimethylformamide/dimethylsulfoxide (1:1) and 50 mL of 10 mM potassium phosphate buffer, pH of 6.8 that contained 0.04% potassium azide. Immobilized thymidine phosphorylase and purine nucleoside phosphorylase (10.7 mL) were added and the suspension mixed on a shaker bath at 37°C. The reaction was monitored by TLC (Silica gel $CHCl_3$:MeOH (9:1). After 24 hours the solids were filtered off and discarded and the filtrate diluted to 300 mL with water. Chromatography was performed by coupling a column containing AG1-X2 hydroxide resin (2.5 x 7 cm) to a column containing XAD-2 resin (2.5 x 11 cm). After sample application, the resins were washed with 1 L of water. Product was eluted with 90% methanol/water (v/v). Further chromatography was performed on a silica gel column (5 x 30 cm). The mobile phase was chloroform/methanol (98:2, v/v). Product containing fractions were combined and after lyophilization yielded 0.17 g that analyzed as a gum containing 0.9 equivalents water (24%): mp <25°C; TLC $R_f$ 0.83 (silica gel, $CHCl_3$:$CH_3OH$/9:1; $[\alpha]_D^{20°}$ = -27.2° (c - 0.5, DMF); UV $\lambda_{max}$ ($\epsilon$ x $10^{-3}$) at pH 7, 287 nm (22.5), 263nm (13.9); at pH 13,287 nm (21.3), 263 nm (13.1).

| Anal. Calcd. for $C_{14}H_{20}N_6O_2 0.9H_2O$: | | | |
|---|---|---|---|
| Calcd.: | C, 52.46; | H, 6.85; | N, 26.22. |
| Found: | C, 52.60; | H, 6.75; | N, 26.15. |

NMR data: $^1$H-NMR (200 MHz, DMSO$d_6$): δ; 7.95 (s, 1H, $H_8$), 6.05 (m, 1H, $H_{1'}$), 5.79 (b, 2H, $NH_2$), 4.99 (t, 1H, J=5.57 Hz, 5′ OH), 4.04 (m, 1H, $H_{4'}$), 3.53 (m, 2H, 5′ $CH_2$), 3.23 (s, 3H, $CH_3$), 3.21 (m, 1H, CH), 2.3 (m, 2H, 2′ $CH_2$), 2.0 (m, 2H, 3′ $CH_2$), and 0.80 and 0.65 (2 multiplets, 2H and 2H, 2-$CH_2$-).

Example 43

2-Amino-6-sec-butoxypurine-9-β-D-2′-3′-dideoxyribofuranoside

2-Amino-6-sec-butoxypurine (0. 5g, 2.4 mmoles) was dissolved in 10ml dimethylforamide and dimethylsulfoxide (1:1). 2′,3′-dideoxythymidien (0.65g, 2.89 mmoles), 30ml of 10mM potassium phosphate buffer, pH 6.8, and purine nucleoside phosphorylase (20000 I.U.) and thymidine phosphorylase (10000 I.U.) immobilized on to DEAE-cellulose were added and the reaction was stirred at 37°C for 2 days. The reaction filtrate was chromatographed on a column of AG1X2 (hydroxide), 2.5 x 9 cm, followed hy a column of XAD-2 (2.5 X 15cm). After applicationof the sample tone liter of water was passed through the columns. The product was eluted with 90% methanol/water. Fractions containing the product were combined and the solvent removed under vacuum. The residue was then chromatographed on a column of silica gel (5 X 20cm) with a mobile phase of methanol/water (9:1). This was followed by chromatography on Sephadex G-10 (5 X 90cm) in 30% n-propanol. After combining the product containing fractions and lyphilization, 0.23g of 2-amino-6-sec-butoxypurine-9-β-D-2′,3′-dideoxyribofuranoside was recovered containing 0.2 water and 0.2 methanol (Melting point = 65°C).
$^1$H-NMR (200 MHz, DMSO-$d_6$) : δ 8.06 (s, 1H, $H_8$), 6.32 (b, 2H, $NH_2$) 6.05 (dd, 1H, $H_{1'}$), 5.32 (m, 1H, CH) 4.93 (t, 1H, J = 5.55 Hz, 5′OH), 4.05 (ml H, $H_{4'}$), 3.5 (m, 2H, 5′ $CH_2$), 2.3 (m, 2H, 2′H), 2.0 (m, 2H, 3′H), 1.65 (m, 1H, $CH_2$), 1.3 (dd, 3H, $CH_3$), 0.89 (t, 3H, $CH_3$).

Example 44

Tablet Formulations

The following formulations A, B and C are prepared by wet granulation of the ingredients with a solution of povidone, followed by addition of magnesium stearate and compression.

| Formulation A | mg/tablet | mg/tablet |
|---|---|---|
| (a) Active ingredient | 250 | 250 |
| (b) Lactose B.P. | 210 | 26 |
| (c) Povidone B.P. | 15 | 9 |
| (d) Sodium Starch Glycollate | 20 | 12 |
| (e) Magnesium Stearate | 5 | 3 |
| | 500 | 300 |

| Formulation B | | |
|---|---|---|
| | mg/tablet | mg/tablet |
| (a) Active ingredient | 250 | 250 |
| (b) Lactose | 150 | - |
| (c) Avicel PH 101 | 60 | 26 |
| (d) Povidone B.P. | 15 | 9 |
| (e) Sodium Starch Glycollate | 20 | 12 |
| (f) Magnesium Stearate | 5 | 3 |
| | 500 | 300 |

| Formulation C | |
|---|---|
| | mg/tablet |
| Active ingredient | 100 |
| Lactose | 200 |
| Starch | 50 |
| Povidone | 5 |
| Magnesium stearate | 4 |
| | 359 |

The following formulations, D and E, are prepared by direct compression of the admixed ingredients. The lactose in formulation E is of the direct compression type (Dairy Crest - "Zeparox").

| Formulation D | |
|---|---|
| | mg/tablet |
| Active ingredient | 250 |
| Pregelatinised Starch NF15 | 150 |
| | 400 |

| Formulation E | |
|---|---|
| | mg/tablet |
| Active ingredient | 250 |
| Lactose | 150 |
| Avicel | 100 |
| | 500 |

Formulation F (Controlled Release Formulation)

The formulation is prepared by wet granulation of the ingredients (below) with a solution of povidone followed by the addition of magnesium stearate and compression.

| | mg/tablet |
|---|---|
| (a) Active ingredient | 500 |
| (b) Hydroxypropylmethylcellulose (Methocel K4M Premium) | 112 |
| (c) Lactose B.P. | 53 |
| (d) Povidone B.P. | 28 |
| (e) Magnesium Stearate | 7 |
| | 700 |

Drug release takes place over a period of about 6-8 hours and is complete after 12 hours.

## Example 45

### Capsule Formulations

#### Formulation A

A capsule formulation is prepared by admixing the ingredients of Formulation D in Example 44 above and filling into a two-part hard gelatin capsule. Formulation B (*infra*) is prepared in a similar manner.

| Formulation B | |
|---|---|
| | mg/capsule |
| (a) Active ingredient | 250 |
| (b) Lactose B.P. | 143 |
| (c) Sodium Starch Glycollate | 25 |
| (d) Magnesium Stearate | 2 |
| | 420 |

| Formulation C | |
|---|---|
| | mg/capsule |
| (a) Active ingredient | 250 |
| (b) Macrogol 4000 B.P. | 350 |
| | 600 |

Capsules of formulation C are prepared by melting the Macrogol 4000 BP, dispersing the active ingredient in the melt and filling the melt into a two-part hard gelatin capsule.

| Formulation D | |
|---|---|
| | mg/capsule |
| Active ingredient | 250 |
| Lecithin | 100 |
| Arachis Oil | 100 |
| | 450 |

Capsules of formulation D are prepared by dispersing the active ingredient in the lecithin and arachis oil and filling the dispersion into soft, elastic gelatin capsules.

Formulation E (Controlled Release Capsule)

The following controlled release capsule formulation is prepared by extruding ingredients a, b and c using an extruder, followed by spheronisation of the extrudate and drying. The dried pellets are then coated with release- controlling membrane (d) and filled into a two-piece, hard gelatin capsule.

| | mg/capsule |
|---|---|
| (a) Active ingredient | 250 |
| (b) Microcrystalline Cellulose | 125 |
| (c) Lactose B.P. | 125 |
| (d) Ethyl Cellulose | 13 |
| | 513 |

Example 46

Injectable Formulation

| Formulation A. | |
|---|---|
| Active ingredient | 0.200g |
| Hydrochloric acid solution, 0.1M, or Sodium hydroxide solution, 0.1M q.s. to pH | 4.0 to 7.0 |
| Sterile water q.s. to | 10ml |

The active ingredient is dissolved in most of the water (35°-40°C) and the pH adjusted to between 4.0 and 7.0 with the hydrochloric acid or the sodium hydroxide as appropriate. The batch is then made up to volume with the water and filtered through a sterile micropore filter into a sterile 10ml amber glass vial (type 1) and sealed with sterile closures and overseals.

| Formulation B. | |
|---|---|
| Active ingredient | 0.125 g |
| Sterile, pyrogen-free, pH 7 phosphate buffer, q.s. to | 25 ml |

Example 47

| Intramuscular injection | |
|---|---|
| Active ingredient | 0.20 g |
| Benzyl Alcohol | 0.10 g |
| Glycofurol 75 | 1.45 g |
| Water for Injection q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml amber glass vials (type 1).

Example 48

| Syrup | |
|---|---|
| Active ingredient | 0.25 g |
| Sorbitol Solution | 1.50 g |
| Glycerol | 2.00 g |
| Sodium Benzoate | 0.005 g |
| Flavour, Peach 17.42.3169 | 0.0125 ml |
| Purified Water q.s. to | 5.00 ml |

The active ingredient is dissolved in a mixture of the glycerol and most of the purified water. An aqueous solution of the sodium benzoate is then added to the solution, followed by addition of the sorbitol solution and finally the flavour. The volume is made up with purified water and mixed well.

Example 49

| Suppository | |
|---|---|
| | mg/suppository |
| Active ingredient (63 m)* | 250 |
| Hard Fat, BP (Witepsol H15 - Dynamit NoBel) | 1770 |
| | 2020 |

*The active ingredient is used as a powder wherein at least 90% of the particles are of 63 m diameter or less.

One-fifth of the Witepsol H15 is melted in a steam-jacketed pan at 45°C maximum. The active ingredient is sifted through a 200 m sieve and added to the molten base with mixing, using a silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250 m stainless steel screen and, with continuous stirring, is allowed to cool to 40°C. At a temperature of 38°C to 40 °C, 2.02g of the mixture is filled into suitable, 2 ml plastic moulds. The suppositories are allowed to cool to room temperature.

Example 50

| Pessaries | |
|---|---|
| | mg/pessary |
| Active ingredient (63 m) | 250 |
| Anhydrate Dextrose | 380 |
| Potato Starch | 363 |
| Magnesium Stearate | 7 |
| | 1000 |

The above ingredients are mixed directly and pessaries prepared by direct compression of the resulting mixture.

## ANTIVIRAL ACTIVITY

### A. Antiviral Activity Against Hepatitis B Virus (HBV)

The human HBV producer cell line of HepG2, 2.2 15, described and characterised by Sells et al ., PNAS 84:1005, 1987 and J. Virol. 62:2836, 1988, has been shown to share many characteristics of the HBV chronically infected hepatocyte. It is infectious as demonstrated by the ability to cause disease in chimpanzees. This cell line was utilised in vitro to identify compounds with anti-HBV activity.

To test compounds for antiviral activity, monolayer cultures were treated with compound, 50-200 µM, for ten days. Supernatant media containing extracellular virion DNA (Dane particles), were harvested on days three, six and ten, treated with proteinase K(1 mg/mL) and sodium dodecyl sulfate (1%), and incubated at 50°C for one hour. DNA was extracted with equal volumes of phenol followed by chloroform and then precipitated by ammonium acetate and propanol. The DNA precipitate was dissolved and collected on nitrocellulose using the procedure of Schieicher and Schuell (S & S, 10 Optical Ave., Keene, NH 03431, Publication #700, 1987), and treated as described by Southern, J. Mol. Biol. 98:503, 1975. Cells were harvested, and the intracellular DNA was obtained after cell lysis with guanidine isothiocyanate. The intracellular DNA was handled in the same manner as the extracellular DNA. After precipitation by ammonium acetate and propanol, the intracellular DNA precipitate was dissolved, cut by restriction endonuclease, Hind III, applied to agarose gel and then treated as described by Southern to determine the quantity of replicative intermediate forms. The antiviral effect of the drug was determined by measuring at least a 100-fold reduction of the amount of Dane particles extruded into the culture and a similar decrease in the intracellular replicative intermediates.

The compound of Example 42, namely 2-amino-6-(cyclopropylmethyl amino)purine-9-β-D-2′,3′-dideoxyribofuranoside showed strong inhibition of HBV at 100 µM.

### B. Antiviral Activity Against Human Immunodeficency Virus (HIV)

Anti-HIV activity was determined by the method of Averett, D.R., J.Virol.Methods , 23 263-276 (1989). The compound of Example 42, namely 2-amino-6(cyclopropylmethylamino)purine-9-β-D-2′,3′-dideoxyribofuranoside, had an average $IC_{50}$ of 17.7 µM (n=7) against HIV-1 and 13.3 µM (n=6) against HIV-2.

## Claims

1. Use of a compound of formula (I)

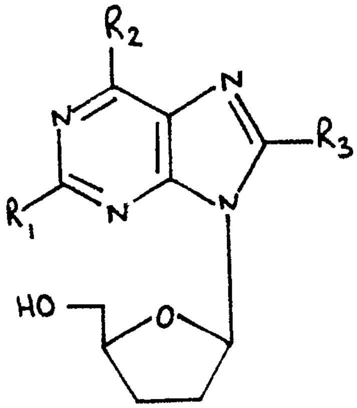

(I)

wherein $R_1$ represents hydrogen or amino; and $R_2$ represents halogen, $C_{1-6}$ alkoxy optionally substituted by $C_{3-6}$ cycloalkyl; $C_{3-8}$ cycloalkyloxy; aryloxy, aralkyl or aralkyloxy in which the aryl may optionally be substituted with lower alkyl, hydroxy or halogen; $C_{3-6}$ cycloalkylthio; $C_{1-6}$ alkylthio; arylthio or aralkylthio in which the aryl may optionally be substituted with lower alkyl, hydroxy, or halogen; or $R_2$ represents a heterocyclic group containing an oxygen atom or one or two nitrogen atoms, and 3-7 carbon atoms with optional double bonds in the ring optionally containing a sulphur and/or oxygen heteroatom and optionally substituted on the ring by one or more lower alkyl, hydroxy or halogen groups, $C_{3-6}$ cycloalkylthio, aralkylthio in which the aryl may be substituted with lower alkyl, hydroxy or halogen; or $R_2$ represents an imidazolylthio group in which the imidazolyl moiety may be substituted with lower alkyl and/or C-substituted with nitro; or $R_2$ represents an amino group which is mono- or di-substituted by $C_{1-6}$ alkyl, $C_{1-6}$ alkoxy, and/or $C_{3-6}$ cycloalkyl, aryl, aralkyl in which the aryl may optionally be substituted with lower alkyl, hydroxy or halogen, allyl optionally substituted with mono- or di-alkyl or alkoxy groups; and $R_3$ represents hydrogen or amino; and pharmaceutically acceptable derivatives thereof, other than a compound of formula (I) in which $R_1$ and $R_3$ represent hydrogen and $R_2$ represents a methoxy or methylthio group, in the manufacture of a pharmaceutical formulation for use in the treatment or prophylaxis of a hepatitis B virus infection.

2. Use of a compound of formula (I) or a pharmaceutically acceptable derivative thereof according to claim 1 wherein $R_1$ represents amino and $R_3$ represents hydrogen.

3. Use of a compound of formula (I) or a pharmaceutically acceptable derivative according to claim 1 or claim 2 wherein $R_2$ represents a mono- or di-substituted amino group.

4. Use of a compound of formula (I) or a pharmaceutically acceptable derivative according to claim 3 wherein the amino group is mono-or di-substituted by $C_{1-6}$ alkyl and/or or $C_{3-6}$ cycloalkyl.

5. Use of compound of formula (I) or a pharmaceutically acceptable derivative thereof according to claim 1 or claim 2 wherein $R_2$ represents a $C_{1-6}$ alkoxy group.

6. Use of compound of formula (I) or pharmaceutically acceptable derivative according to claim 1 wherein said compound is selected from the following:-

2-amino-6-n-propoxypurine-9-β-D-2′,3′-dideoxyribofuranoside; and

2-amino-6-cyclopropylaminopurine-9-β-D-2′,3′-dideoxyribofuranoside

7. 2-Amino-6-(cyclopropylmethylamino)purine-9-β-D-2′,3′-dideoxyribofuranoside and pharmaceutically acceptable derivatives thereof.

8. Compounds according to claim 7 for use in medical therapy.

9. Compounds according to claim 8 for use in the treatment or prophylaxis of a human retrovirus infection.

10. A compound according to claim 9 for use in the treatment or prophylaxis of a Human Immunodeficiency Virus (HIV) infection.

11. A compound according to claim 8 for use in the treatment or prophylaxis of a Hepatitis B Virus (HBV) infection.

12. Pharmaceutical formulations comprising at least one compound as claimed in claim 7 together with at least one pharmaceutically acceptable carrier therefor.

13. A process for the preparation of 2-amino-6-(cyclopropylmethylamino)purine9-β-D-ribofurnoside and pharmaceutically acceptable derivatives thereof which comprises

(a) reacting a compound of formula (II)

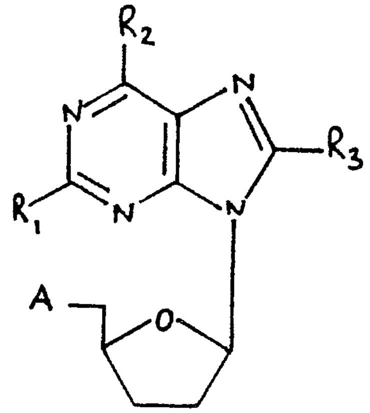

(II)

(wherein $R_1$ represents amino, $R_2$ represents cyclopropylmethylamino, $R_3$ represents hydrogen and A represents a precursor group for the hydroxy group), with an agent or under conditions to convert said precursor group into the desired group; or

(b) reacting a purine base of formula (II)

B-H (III)

wherein B is a purine base according to claim 1 wherein $R_1$, $R_2$ and $R_3$ are as defined above or a functional equivalent thereof, with a compound serving to introduce the desired dideoxyribofuranosyl ring at the 9-position of the purine base of formula (III);

and thereafter, or simultaneously therewith, effecting one or more of the following optional conversions:-

(i) when a the desired parent compound is formed, converting it into a pharmaceutically acceptable derivative thereof.

(ii) when a pharmaceutically acceptable derivative of the desired parent compound formed, converting the said derivative into the parent compound, or a different derivative thereof.

Claims for the following Contracting States: ES, GR

1. A process for the preparation of a compound of formula (I)

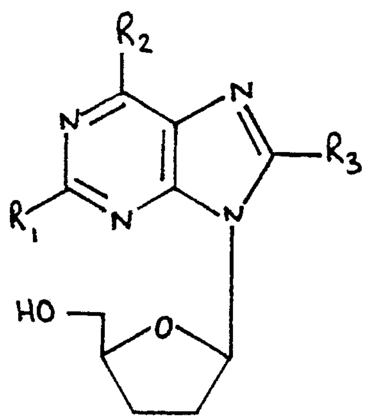

(I)

wherein $R_1$ represents hydrogen or amino; and $R_2$ represents halogen, $C_{1-6}$ alkoxy optionally substituted by $C_{3-6}$ cycloalkyl; $C_{3-8}$ cycloalkyloxy; aryloxy, aralkyl or aralkyloxy in which the aryl may optionally be substituted with lower alkyl, hydroxy or halogen; $C_{3-6}$ cycloalkylthio; $C_{1-6}$ alkylthio; arylthio or aralkylthio in which the aryl may optionally be substituted with lower alkyl, hydroxy, or halogen; or $R_2$ represents a heterocyclic group containing an oxygen atom or one or two nitrogen atoms, and 3-7 carbon atoms with optional double bonds in the ring optionally containing a sulphur and/or oxygen heteroatom and optionally substituted on the ring by one or more lower alkyl, hydroxy or halogen groups, $C_{3-6}$ cycloalkylthio, aralkylthio in which the aryl may be substituted with lower alkyl, hydroxy or halogen; or $R_2$ represents an imidazolylthio group in which the imidazolyl moiety may be substituted with lower alkyl and/or C-substituted with nitro; or $R_2$ represents an amino group which is mono- or di-substituted by $C_{1-6}$ alkyl, $C_{1-6}$ alkoxy,

and/or $C_{3-6}$ cycloalkyl, aryl, aralkyl in which the aryl may optionally be substituted with lower alkyl, hydroxy or halogen, allyl optionally substituted with mono- or di-alkyl or alkoxy groups; and $R_3$ represents hydrogen or amino; and pharmaceutically acceptable derivatives thereof, other than a compound of formula (I) in which $R_1$ and $R_3$ represent hydrogen and $R_2$ represents a methoxy or methylthio group, which comprises
(a) reacting a compound of formula (II)

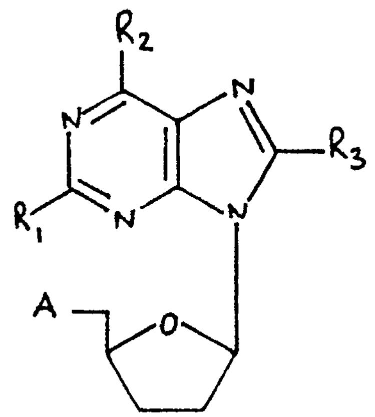

(II)

(wherein $R_1$, $R_2$ and $R_3$ are as defined above and A represents a precursor group for the hydroxy group), with an agent or under conditions to convert said precursor group into the desired group; or
(b) reacting a purine base of formula (II)
B-H (III)
wherein B is a purine base according to claim 1 wherein $R_1$, $R_2$ and $R_3$ are as defined above or a functional equivalent thereof, with a compound serving to introduce the desired dideoxyribofuranosyl ring at the 9-position of the purine base of formula (III);
and thereafter, or simultaneously therewith, effecting one or more of the following optional conversions:-
(i) when a the desired parent compound is formed, converting it into a pharmaceutically acceptable derivative thereof.
(ii) when a pharmaceutically acceptable derivative of the desired parent compound formed, converting the said derivative into the parent compound, or a different derivative thereof; and subsequently converting the said compound of formula (I) or a pharmaceutically acceptable derivative thereof into a pharmaceutical formulation for the treatment or prophylaxis of a hepatitis B virus infection.
2. A process for the preparation of 2-amino-6-(cyclopropylmethylamino)purine-9-β-D-ribofurnoside and pharmaceutically acceptable derivatives thereof which comprises
(a) reacting a compound of formula (II)

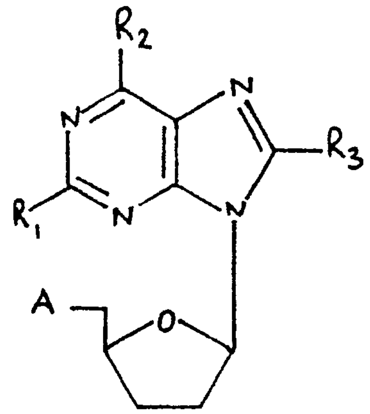

(II)

(wherein $R_1$ represents amino, $R_2$ represents cyclopropylmethylamino, $R_3$ represents hydrogen and A represents a precursor group for the hydroxy group), with an agent or under conditions to convert said precursor group into the desired group; or
(b) reacting a purine base of formula (II)

B-H (III)

wherein B is a purine base according to claim 1 wherein $R_1$, $R_2$ and $R_3$ are as defined above or a functional equivalent thereof, with a compound serving to introduce the desired dideoxyribofura nosyl ring at the 9-position of the purine base of formula (III);

and thereafter, or simultaneously therewith, effecting one or more of the following optional conversions:-

(i) when a the desired parent compound is formed, converting it into a pharmaceutically acceptable derivative thereof.

(ii) when a pharmaceutically acceptable derivative of the desired parent compound formed, converting the said derivative into the parent compound, or a different derivative thereof.

European Patent Office

# EUROPEAN SEARCH REPORT

Application Number

**EP 90 31 0965**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 206 497 (THE WELLCOME FOUNDATION LTD)<br>* The whole document * | 1-13 | C 07 D 405/04<br>A 61 K 31/70 |
| Y | EP-A-0 002 192 (THE WELLCOME FOUNDATION LTD)<br>* Page 1, lines 6-10; claim 1 * | 1-13 | |
| Y | EP-A-0 217 580 (THE WELLCOME FOUNDATION LTD)<br>* Page 1, line 1 - page 4, line 3; page 10, lines 12-21 * | 1-13 | |
| A | TETRAHEDRON LETTERS, vol. 21, no. 45, 1980, pages 4339-4342, Pergamon Press Ltd, GB; J. ENGELS: "Synthesis of 2'-end modified 2',5'-adenylate trimers"<br>* Compound 9 * | 1 | |
| P,A | CHEMICAL ABSTRACTS, vol. 110, no. 3, 16th January 1989, page 71, abstract no. 18631f, Columbus, Ohio, US; M.J. LOHSE et al.: "2',3'-Dedeoxy-N6-cyclohexyladenosine: an adenosine derivative with antagonist properties at adenosine receptors",<br>& EUR. J. PHARMACOL. 1988, 156(1), 157-60<br>* Abstract * | 1 | |

TECHNICAL FIELDS SEARCHED (Int. Cl.5)

C 07 D 405/00
A 61 K 31/00

The present search report has been drawn up for all claims

| Place of search | Date of completion of search | Examiner |
|---|---|---|
| The Hague | 07 December 90 | SCOTT J.R.M. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document